# EUROPEAN PATENT SPECIFICATION

(11) **EP 2 756 085 B1**
(45) Date of publication and mention of the grant of the patent: **20.03.2019**
(21) Application number: 12831567.8
(22) Date of filing: 12.09.2012
(51) Int. Cl.: C12N 15/82

(54) **METHODS AND COMPOSITIONS FOR WEED CONTROL**
VERFAHREN UND ZUSAMMENSETZUNGEN ZUR UNKRAUTBEKÄMPFUNG
PROCÉDÉS ET COMPOSITIONS DE LUTTE CONTRE LES MAUVAISES HERBES

(30) Priority: 13.09.2011 US 201161534057 P
(43) Date of publication of application: 23.07.2014
(73) Proprietor: Monsanto Technology LLC, St. Louis, MO 63167 (US)
(72) Inventor: SHAH, Ronak, Hasmukh, St. Louis, MO 63167 (US); WANG, Dafu, St. Louis, MO 63167 (US); LI, Zhaolong, St. Louis, MO 63167 (US); ADER, Daniel, St. Louis, MO 63167 (US); TAO, Nengbing, St. Louis, MO 63167 (US); YANG, Heping, St. Louis, MO 63167 (US)
(74) Representative: Uexküll & Stolberg
(86) International application number: PCT/US2012/054789
(87) International publication number: WO 2013/039990

(56) References cited:
- WO-A1-2011/112570
- WO-A2-03/077648
- US-A1- 2006 009 358
- US-A1- 2006 135 758
- JONATHAN GRESSEL ET AL: "A strategy to provide long-term control of weedy rice while mitigating herbicide resistance transgene flow, and its potential use for other crops with related weeds", PEST MANAGEMENT SCIENCE, vol. 65, no. 7, 1 July 2009 (2009-07-01), pages 723-731, XP055053395, ISSN: 1526-498X, DOI: 10.1002/ps.1754
- MONSANTO: "Do Monsanto have the next big thing?", INTERNET CITATION, 23 April 2013 (2013-04-23), XP007922963, Retrieved from the Internet: URL:http://ahri.uwa.edu.au/do-monsanto-hav e-the-next-big-thing/ [retrieved on 2015-01-19]
- HOFGEN ET AL.: 'Repression of Acetolactate Synthase Activity through Antisense Inhibition 0 (Molecular and Biochemical Analysis of Transgenic Potato (Solanum tuberosum L. cv Desiree) Plants).' PLANT PHYSIOL vol. 107, no. 2, February 1995, pages 469 - 477, XP002145367
- ZHANG ET AL.: 'Agrobacterium-mediated transformation of Arabidopsis thaliana using the floral dip method.' NATURE PROTOCOLS vol. 1, no. 2, 2006, pages 469 - 477, XP055146758
- VENCILL ET AL.: 'Resistance of Weeds to Herbicides.' HERBICIDES AND ENVIRONMENT. 08 January 2011, NEW YORK, pages 585 - 594, XP055146760 ISBN: 978-953-307-4 Retrieved from the Internet: <URL:http://www.intechopen.com/books/herbic ides-and-environment/resistance-of-weeds-to - herbicides>

## Description

### FIELD

The methods and compositions generally relates to the field of weed management. More specifically, relates to 5-enolpyruvylshikimate-3-phosphate synthase genes in plants and compositions containing polynucleotide molecules for modulating and/or regulating their expression. Further provided are methods and compositions useful for weed control.

### BACKGROUND

Weeds are plants that compete with cultivated plants in an agronomic environment and cost farmers billions of dollars annually in crop losses and the expense of efforts to keep weeds under control. Weeds also serve as hosts for crop diseases and insect pests. The losses caused by weeds in agricultural production environments include decreases in crop yield, reduced crop quality, increased irrigation costs, increased harvesting costs, reduced land value, injury to livestock, and crop damage from insects and diseases harbored by the weeds. The principal means by which weeds cause these effects are: 1) competing with crop plants for water, nutrients, sunlight and other essentials for growth and development, 2) production of toxic or irritant chemicals that cause human or animal health problem, 3) production of immense quantities of seed or vegetative reproductive parts or both that contaminate agricultural products and perpetuate the species in agricultural lands, and 4) production on agricultural and nonagricultural lands of vast amounts of vegetation that must be disposed of. Herbicide tolerant weeds are a problem with nearly all herbicides in use, there is a need to effectively manage these weeds. There are over 365 weed biotypes currently identified as being herbicide resistant to one or more herbicides by the Herbicide Resistance Action Committee (HRAC), the North American Herbicide Resistance Action Committee (NAHRAC), and the Weed Science Society of America (WSSA).

The EPSPS (5-enolpyruvylshikimate-3-phosphate synthase) enzyme catalyzes the conversion of shikimate-3-phosphate into 5-enolpyruvyl-shikimate-3-phosphate, an intermediate in the biochemical pathway for creating three essential aromatic amino acids (tyrosine, phenylalanine, and tryptophan). The EPSPS enzyme is the target for the herbicide N-phosphonomethyl glycine also known as glyphosate.

### SUMMARY

In one aspect the present invention provides a method of plant control comprising: topically applying to a surface of a plant a composition comprising a double-stranded RNA (dsRNA) polynucleotide and a transfer agent, wherein said dsRNA polynucleotide is identical or complementary to an RNA sequence of a 5-enolpyruvylshikimate-3-phosphate synthase (EPSPS) gene sequence selected from the group consisting of SEQ ID NOs: 11, 18, 19, 21, 22, 53-55, 61, 72, 74, 75, 84, 88, 99, 101, 110, 115, 119, and an at least 18 contiguous nucleotides polynucleotide fragment thereof, wherein said transfer agent is an organosilicone surfactant composition or an organosilicone compound contained therein and conditions said surface of said plant for permeation by said dsRNA polynucleotide, and whereby said plant's growth, development, or reproductive ability is reduced or said plant is more sensitive to an EPSPS inhibitor herbicide relative, to an untreated plant.

In a further aspect the present invention provides a composition for topical application to a surface of a plant comprising a dsRNA polynucleotide and a transfer agent, wherein said dsRNA polynucleotide is identical or complementary to an RNA sequence of an EPSPS gene sequence selected from the group consisting of SEQ ID NOs: 11, 18, 19, 21, 22, 53-55, 61, 72, 74, 75, 84, 88, 99, 101, 110, 115, 119, and an at least 18 contiguous nucleotides polynucleotide fragment thereof, wherein said transfer agent is an organosilicone surfactant composition or an organosilicone compound contained therein and conditions said surface of said plant for permeation by said dsRNA polynucleotide, and whereby said plant treated with said composition has its growth, development, or reproductive ability suppressed or delayed or said plant is more sensitive to an EPSPS inhibitor herbicide as a result of said dsRNA polynucleotide containing composition, relative to an untreated plant.

In a further embodiment the present invention provides a method of reducing expression of an EPSPS gene in a plant comprising: topically applying to a surface of a plant a composition comprising a dsRNA polynucleotide and a transfer agent, wherein said dsRNA polynucleotide is identical or complementary to an RNA sequence of an EPSPS gene sequence selected from the group consisting of SEQ ID NOs: 11, 18, 19, 21, 22, 53-55, 61, 72, 74, 75, 84, 88, 99, 101, 110, 115, 119, and an at least 18 contiguous nucleotides polynucleotide fragment thereof, wherein said transfer agent is an organosilicone surfactant composition or an organosilicone compound contained therein and conditions the surface of said plant for permeation by said dsRNA polynucleotide, and whereby said expression of said EPSPS gene is reduced, relative to an untreated plant.

The present invention also provides a method of identifying dsRNA polynucleotides useful in modulating EPSPS gene expression when topically applied to a surface of a plant comprising: a) providing a plurality of dsRNA polynucleotides that comprise a region identical or complementary to at least 18 contiguous nucleotides of an RNA sequence of an EPSPS gene sequence selected from the group consisting of SEQ ID NOs: 11, 18, 19, 21, 22, 53-55, 61, 72, 74, 75, 84, 88, 99, 101, 110, 115, and 119; b) topically applying to said surface of said plant a composition comprising one or more of said dsRNA polynucleotides and a transfer agent; and c) analyzing said plant, or a plant extract thereof, for modulation of EPSPS gene expression, wherein said transfer agent is an organosilicone surfactant composition or an organosilicone compound contained therein and conditions the surface of said plant for permeation by said dsRNA polynucleotide, and whereby said plant treated with said composition has its growth, development, or reproductive ability suppressed or delayed or said plant is more sensitive to an EPSPS inhibitor herbicide as a result of said dsRNA polynucleotide containing composition, relative to an untreated plant.

In a separate aspect the present invention provides an agricultural chemical composition for topical application to a surface of a plant comprising an admixture of a dsRNA polynucleotide, a transfer agent, glyphosate, and a co-herbicide, wherein said dsRNA polynucleotide is identical or complementary to an RNA sequence of an EPSPS gene sequence selected from the group consisting of SEQ ID NOs: 11, 18, 19, 21, 22, 53-55, 61, 72, 74, 75, 84, 88, 99, 101, 110, 115, 119, and an at least 18 contiguous nucleotides polynucleotide fragment thereof, wherein said transfer agent is an organosilicone surfactant composition or an organosilicone compound contained therein and conditions the surface of said plant for permeation by said dsRNA polynucleotide and whereby said plant treated with said composition has its growth, development, or reproductive ability suppressed or delayed or said plant is more sensitive to an EPSPS inhibitor herbicide as a result of said dsRNA polynucleotide containing composition, relative to an untreated plant.

In a further aspect the present invention provides an agricultural chemical composition for topical application to a surface of a plant comprising an admixture of a dsRNA polynucleotide, a transfer agent glyphosate, and a pesticide, wherein said dsRNA polynucleotide is identical or complementary to an RNA sequence of an EPSPS gene sequence selected from the group consisting of SEQ ID NOs: 11, 18, 19, 21, 22, 53-55, 61, 72, 74, 75, 84, 88, 99, 101, 110, 115, 119, and an at least 18 contiguous nucleotides polynucleotide fragment thereof, wherein said transfer agent is an organosilicone surfactant composition or an organosilicone compound contained therein and conditions the surface of said plant for permeation by said dsRNA polynucleotide and whereby said plant treated with said composition has its growth, development, or reproductive ability suppressed or delayed or said plant is more sensitive to an EPSPS inhibitor herbicide as a result of said dsRNA polynucleotide containing composition, relative to an untreated plant.

### BRIEF DESCRIPTION OF THE FIGURES

The following drawings form part of the present specification and are included to further demonstrate certain aspects of the function of the compositions and method. The function may be better understood by reference to one or more of these drawings in combination with the detailed description of specific embodiments presented herein. The function can be more fully understood from the following description of the figures:
Figure 1. Regions of the Palmer amaranth EPSPS coding sequence that are sensitive to trigger molecules
Figure 2. Transgenic glyphosate tolerant corn plants treated with trigger polynucleotides and glyphosate
Figure 3. Transgenic cotton plants treated with trigger polynucleotides and glyphosate

### DETAILED DESCRIPTION

Provided are methods and compositions containing a polynucleotide that provide for regulation, repression or delay and/or modulation of EPSPS (5-enolpyruvylshikimate-3-phosphate synthase) gene expression and enhanced control of weedy plant species and importantly glyphosate resistant weed biotypes. Aspects of the method can be applied to manage various weedy plants in agronomic and other cultivated environments.

The following definitions and methods are provided to guide those of ordinary skill in the art. Unless otherwise noted, terms are to be understood according to conventional usage by those of ordinary skill in the relevant art. Where a term is provided in the singular, the inventors also contemplate aspects described by the plural of that term.

By "non-transcribable" polynucleotides is meant that the polynucleotides do not comprise a complete polymerase II transcription unit.

As used herein "solution" refers to homogeneous mixtures and non-homogeneous mixtures such as suspensions, colloids, micelles, and emulsions.

Weedy plants are plants that compete with cultivated plants, those of particular importance include, but are not limited to important invasive and noxious weeds and herbicide resistant biotypes in crop production, such as, *Amaranthus* species *-A. albus, A. blitoides, A. hybridus, A. palmeri, A. powellii, A. retroflexus, A. spinosus, A. tuberculatus,* and *A. viridis; Ambrosia* species - *A. trifida, A. artemisifolia; Lolium* species *-L. multiflorum, L. rigidium, L perenne; Digitaria* species *-D. insularis; Euphorbia* species *-E. heterophylla; Kochia species* - *K. scoparia; Sorghum* species *-S. halepense; Conyza* species *-C. bonariensis, C. canadensis, C. sumatrensis; Chloris* species *-C. truncate; Echinochola* species - *E. colona, E. crus-galli; Eleusine species -E. indica; Poa* species *-P. annua; Plantago* species *-P. lanceolata; Avena species* - *A. fatua; Chenopodium* species - *C*. *album; Setaria* species - *S. viridis, Abutilon theophrasti, Ipomoea* species, *Sesbania,* species, *Cassia* species, *Sida* species, *Brachiaria,* species and *Solanum* species.

Additional weedy plant species found in cultivated areas include *Alopecurus myosuroides, Avena sterilis, Avena sterilis ludoviciana, Brachiaria plantaginea, Bromus diandrus, Bromus rigidus, Cynosurus echinatus, Digitaria ciliaris, Digitaria ischaemum, Digitaria sanguinalis, Echinochloa oryzicola, Echinochloa phyllopogon, Eriochloa punctata, Hordeum glaucum, Hordeum leporinum, Ischaemum rugosum, Leptochloa chinensis, Lolium persicum,*, *Phalaris minor, Phalaris paradoxa, Rottboellia exalta, Setaria faberi, Setaria viridis var, robusta-alba schreiber, Setaria viridis var, robusta -purpurea, Snowdenia polystachea, Sorghum sudanese, Alisma plantago-aquatica, Amaranthus lividus, Amaranthus quitensis, Ammania auriculata, Ammania coccinea, Anthemis cotula, Apera spica-venti, Bacopa rotundifolia, Bidens pilosa, Bidens subalternans, Brassica tournefortii, Bromus tectorum, Camelina microcarpa, Chrysanthemum coronarium, Cuscuta campestris, Cyperus difformis, Damasonium minus, Descurainia sophia, Diplotaxis tenuifolia, Echium plantagineum, Elatine triandra var, pedicellata, Euphorbia heterophylla, Fallopia convolvulus, Fimbristylis miliacea, Galeopsis tetrahit, Galium spurium, Helianthus annuus, Iva xanthifolia, Ixophorus unisetus, Ipomoea indica, Ipomoea purpurea, Ipomoea sepiaria, Ipomoea aquatic, Ipomoea triloba, Lactuca serriola, Limnocharis flava, Limnophila erecta, Limnophila sessiliflora, Lindernia dubia, Lindernia dubia var, major, Lindernia micrantha, Lindernia procumbens, Mesembryanthemum crystallinum, Monochoria korsakowii, Monochoria vaginalis, Neslia paniculata, Papaver rhoeas, Parthenium hysterophorus, Pentzia suffruticosa, Phalaris minor, Raphanus raphanistrum, Raphanus sativus, Rapistrum rugosum, Rotala indica var, uliginosa, Sagittaria guyanensis, Sagittaria montevidensis, Sagittaria pygmaea, Salsola iberica, Scirpus juncoides var, ohwianus, Scirpus mucronatus, Setaria lutescens, Sida spinosa, Sinapis arvensis, Sisymbrium orientale, Sisymbrium thellungii, Solanum ptycanthum, Sonchus asper, Sonchus oleraceus, Sorghum bicolor, Stellaria media, Thlaspi arvense, Xanthium strumarium, Arctotheca calendula, Conyza sumatrensis, Crassocephalum crepidiodes, Cuphea carthagenenis, Epilobium adenocaulon, Erigeron philadelphicus, Landoltia punctata, Lepidium virginicum, Monochoria korsakowii, Solanum americanum, Solanum nigrum, Vulpia bromoides, Youngia japonica, Hydrilla verticillata, Carduus nutans, Carduus pycnocephalus, Centaurea solstitialis, Cirsium arvense, Commelina diffusa, Convolvulus arvensis, Daucus carota, Digitaria ischaemum, Echinochloa crus-pavonis, Fimbristylis miliacea, Galeopsis tetrahit, Galium spurium, Limnophila erecta, Matricaria perforate, Papaver rhoeas, Ranunculus acris, Soliva sessilis, Sphenoclea zeylanica, Stellaria media, Nassella trichotoma, Stipa neesiana, Agrostis stolonifera, Polygonum aviculare, Alopecurus japonicus, Beckmannia syzigachne, Bromus tectorum, Chloris inflate, Echinochloa erecta, Portulaca oleracea, and Senecio vulgaris.* It is believed that all plants contain a phytoene desaturase gene in their genome, the sequence of which can be isolated and polynucleotides made according to the methods of the present invention that are useful for regulation, suppressing or delaying the expression of the target EPSPS gene in the plants and the growth or development of the treated plants.

A cultivated plant may also be considered a weedy plant when it occurs in unwanted environments. For example, corn plants growing in a soybean field. Transgenic crops with one or more herbicide tolerances may need specialized methods of management to control weeds and volunteer crop plants. The method enables the targeting of a transgene for herbicide tolerance to permit the treated plants to become sensitive to the herbicide. For example, an EPSPS DNA contained in a transgenic crop event can be a target for trigger molecules in order to render the transgenic crop sensitive to application of the corresponding glyphosate containing herbicide. Such transgenic events are known in the art and include but are not limited to DAS-44406-6, MON883302, MON87427, FG72, HCEM485, H7-1, ASR368, J101, J163, DP-098140, GHB614, 356043, MON89788, MON88913, RT200, NK603, GTSB77, GA21, MON1445, and 40-3-2 and US patent publications: 20110126310, 20090137395

A "trigger" or "trigger polynucleotide" is a polynucleotide molecule that is homologous or complementary to a target gene polynucleotide. The trigger polynucleotide molecules modulate expression of the target gene when topically applied to a plant surface with a transfer agent, whereby a plant treated with said composition has its growth or development or reproductive ability regulated, suppressed or delayed or said plant is more sensitive to an EPSPS inhibitor herbicide as a result of said polynucleotide containing composition relative to a plant not treated with a composition containing the trigger molecule. Trigger polynucleotides disclosed herein are generally described in relation to the target gene sequence and maybe used in the sense (homologous) or antisense (complementary) orientation as single stranded molecules or comprise both strands as double stranded molecules or nucleotide variants and modified nucleotides thereof depending on the various regions of a gene being targeted.

It is contemplated that the composition may contain multiple polynucleotides and herbicides that include but are not limited to EPSPS gene trigger polynucleotides and an EPSPS inhibitor herbicide and one or more additional herbicide target gene trigger polynucleotides and the related herbicides and one or more additional essential gene trigger polynucleotides. Essential genes are genes in a plant that provide key enzymes or other proteins, for example, a biosynthetic enzyme, metabolizing enzyme, receptor, signal transduction protein, structural gene product, transcription factor, or transport protein; or regulating RNAs, such as, microRNAs, that are essential to the growth or survival of the organism or cell or involved in the normal growth and development of the plant (Meinke, et al., Trends Plant Sci. 2008:13(9):483-91). The suppression of an essential gene enhances the effect of a herbicide that affects the function of a gene product different than the suppressed essential gene. The compositions can include various trigger polynucleotides that modulate the expression of an essential gene other than an EPSPS gene.

Herbicides for which transgenes for plant tolerance have been demonstrated and the method can be applied, include but are not limited to: auxin-like herbicides, glyphosate, glufosinate, sulfonylureas, imidazolinones, bromoxynil, delapon, dicamba, cyclohezanedione, protoporphyrionogen oxidase inhibitors, 4-hydroxyphenyl-pyruvate-dioxygenase inhibitors herbicides. For example, transgenes and their polynucleotide molecules that encode proteins involved in herbicide tolerance are known in the art, and include, but are not limited to an 5-enolpyruvylshikimate-3-phosphate synthase (EPSPS), for example, as more fully described in U.S. Pat. Nos. 7,807,791 (SEQ ID NO:5); 6,248,876 B1; 5,627,061; 5,804,425; 5,633,435; 5,145,783; 4,971,908; 5,312,910; 5,188,642; 4,940,835; 5,866,775; 6,225,114 B1; 6,130,366; 5,310,667; 4,535,060; 4,769,061; 5,633,448; 5,510,471; U.S. Pat. No. Re. 36,449; U.S. Pat. Nos. RE 37,287 E; and 5,491,288; tolerance to sulfonylurea and/or imidazolinone, for example, as described more fully in U.S. Pat. Nos. 5,605,011; 5,013,659; 5,141,870; 5,767,361; 5,731,180; 5,304,732; 4,761,373; 5,331,107; 5,928,937; and 5,378,824; and international publication WO 96/33270; tolerance to hydroxyphenylpyruvatedioxygenases inhibiting herbicides in plants are described in U.S. Pat. Nos. 6,245,968 B1; 6,268,549; and 6,069,115; and US7,312,379 SEQ ID NO:3; US7,935,869; US7,304,209, SEQ ID NO:1, 3,5 and 15; aryloxyalkanoate dioxygenase polynucleotides, which confer tolerance to 2,4-D and other phenoxy auxin herbicides as well as to aryloxyphenoxypropionate herbicides as described, for example, in WO2005/107437; US7,838,733 SEQ ID NO:5;) and dicamba-tolerance polynucleotides as described, for example, in Herman et al. (2005) J. Biol. Chem. 280: 24759-24767. Other examples of herbicide-tolerance traits include those conferred by polynucleotides encoding an exogenous phosphinothricin acetyltransferase, as described in U.S. Pat. Nos. 5,969,213; 5,489,520; 5,550,318; 5,874,265; 5,919,675; 5,561,236; 5,648,477; 5,646,024; 6,177,616; and 5,879,903. Plants containing an exogenous phosphinothricin acetyltransferase can exhibit improved tolerance to glufosinate herbicides, which inhibit the enzyme glutamine synthase. Additionally, herbicide-tolerance polynucleotides include those conferred by polynucleotides conferring altered protoporphyrinogen oxidase (protox) activity, as described in U.S. Pat. Nos. 6,288,306 B1; 6,282,837 B1; and 5,767,373; and WO 01/12825. Plants containing such polynucleotides can exhibit improved tolerance to any of a variety of herbicides which target the protox enzyme (also referred to as protox inhibitors). Polynucleotides encoding a glyphosate oxidoreductase and a glyphosate-N-acetyl transferase (GOX described in U.S. Patent 5,463,175 and GAT described in U.S. Patent publication 20030083480, dicamba monooxygenase U.S. Patent 7,022,896 and 7,884,262 ); a polynucleotide molecule encoding bromoxynil nitrilase (*Bxn* described in U.S. Patent No. 4,810,648 for Bromoxynil tolerance ); a polynucleotide molecule encoding phytoene desaturase (c*rtI*) described in Misawa et al, (1993) Plant J. 4:833-840 and Misawa et al, (1994) Plant J. 6:481-489 for norflurazon tolerance; a polynucleotide molecule encoding acetohydroxyacid synthase (AHAS, *aka* ALS) described in Sathasiivan et al. (1990) Nucl. Acids Res. 18:2188-2193 for tolerance to sulfonylurea herbicides; and the *bar* gene described in DeBlock, et al. (1987) EMBO J. 6:2513-2519 for glufosinate and bialaphos tolerance. The transgenic coding regions and regulatory elements of the herbicide tolerance genes are targets in which polynucleotide triggers and herbicides can be included in the composition and combinations thereof to provide for enhanced methods of weed control.

"Glyphosate" (N-phosphonomethylglycine) herbicide inhibits the shikimic acid pathway which leads to the biosynthesis of aromatic compounds including amino acids, plant hormones and vitamins. Specifically, glyphosate curbs the conversion of phosphoenolpyruvic acid (PEP) and 3-phosphoshikimic acid to 5-enolpyruvyl-3-phosphoshikimic acid by inhibiting the enzyme 5-enolpyruvylshikimate-3-phosphate synthase (hereinafter referred to as EPSP synthase or EPSPS). The term "glyphosate" should be considered to include any herbicidally effective form of N-phosphonomethylglycine (including any salt thereof) and other forms which result in the production of the glyphosate anion in planta. Glyphosate is an example of an EPSPS inhibitor herbicide. Herbicides are molecules that affect plant growth or development or reproductive ability.

Glyphosate is commercially available in numerous formulations. Examples of these formulations of glyphosate include, without limitation, those sold by Monsanto Company (St Louis, MO) as ROUNDUP.RTM., ROUNDUP.RTM. ULTRA, ROUNDUP.RTM. ULTRAMAX, ROUNDUP.RTM. CT, ROUNDUP.RTM. EXTRA, ROUNDUP.RTM. BIACTIVE, ROUNDUP.RTM. BIOFORCE, RODEO.RTM., POLARIS.RTM., SPARK.RTM. and ACCORD.RTM. herbicides, all of which contain glyphosate as its isopropylammonium salt, ROUNDUP.RTM. WEATHERMAX containing glyphosate as its potassium salt; ROUNDUP.RTM. DRY and RIVAL.RTM. herbicides, which contain glyphosate as its ammonium salt; ROUNDUP.RTM. GEOFORCE, which contains glyphosate as its sodium salt; and TOUCHDOWN.RTM. herbicide (Syngenta, Greensboro, NC), which contains glyphosate as its trimethylsulfonium salt. Various other salts of glyphosate are available for example, dimethylamine salt, isopropylamine salt, trimesium salt, potassium salt, monoammonium salt, and diammonium salt. Commerical formulations and application rates thereof are often defined in terms of acid equivalent pounds pe acre (a.e. Lb/ac).

Numerous herbicides with similar or different modes of action (herein referred to as co-herbicides) are available that can be added to the composition that provide multi-species weed control or alternative modes of action for difficult to control weed species, for example, members of the herbicide families that include but are not limited to amide herbicides, aromatic acid herbicides, arsenical herbicides, benzothiazole herbicides, benzoylcyclohexanedione herbicides, benzofuranyl alkylsulfonate herbicides, carbamate herbicides, cyclohexene oxime herbicides, cyclopropylisoxazole herbicides, dicarboximide herbicides, dinitroaniline herbicides, dinitrophenol herbicides, diphenyl ether herbicides, dithiocarbamate herbicides, halogenated aliphatic herbicides, imidazolinone herbicides, inorganic herbicides, nitrile herbicides, organophosphorus herbicides, oxadiazolone herbicides, oxazole herbicides, phenoxy herbicides, phenylenediamine herbicides, pyrazole herbicides, pyridazine herbicides, pyridazinone herbicides, pyridine herbicides, pyrimidinediamine herbicides, pyrimidinyloxybenzylamine herbicides, quaternary ammonium herbicides, thiocarbamate herbicides, thiocarbonate herbicides, thiourea herbicides, triazine herbicides, triazinone herbicides, triazole herbicides, triazolone herbicides, triazolopyrimidine herbicides, uracil herbicides, and urea herbicides. In particular, the rates of use of the added herbicides can be reduced in compositions comprising the polynucleotides. Use rate reductions of the additional added herbicides can be 10-25 percent, 26-50 percent, 51-75 percent or more can be achieved that enhance the activity of the polynucleotides and herbicide composition and is contemplated. Representative herbicides of the families include but are not limited to acetochlor, acifluorfen, acifluorfen-sodium, aclonifen, acrolein, alachlor, alloxydim, allyl alcohol, ametryn, amicarbazone, amidosulfuron, aminopyralid, amitrole, ammonium sulfamate, anilofos, asulam, atraton, atrazine, azimsulfuron, BCPC, beflubutamid, benazolin, benfluralin, benfuresate, bensulfuron, bensulfuron-methyl, bensulide, bentazone, benzfendizone, benzobicyclon, benzofenap, bifenox, bilanafos, bispyribac, bispyribac-sodium, borax, bromacil, bromobutide, bromoxynil, butachlor, butafenacil, butamifos, butralin, butroxydim, butylate, cacodylic acid, calcium chlorate, cafenstrole, carbetamide, carfentrazone, carfentrazone-ethyl, CDEA, CEPC, chlorflurenol, chlorflurenol-methyl, chloridazon, chlorimuron, chlorimuron-ethyl, chloroacetic acid, chlorotoluron, chlorpropham, chlorsulfuron, chlorthal, chlorthal-dimethyl, cinidon-ethyl, cinmethylin, cinosulfuron, cisanilide, clethodim, clodinafop, clodinafop-propargyl, clomazone, clomeprop, clopyralid, cloransulam, cloransulam-methyl, CMA, 4-CPB, CPMF, 4-CPP, CPPC, cresol, cumyluron, cyanamide, cyanazine, cycloate, cyclosulfamuron, cycloxydim, cyhalofop, cyhalofop-butyl, 2,4-D, 3,4-DA, daimuron, dalapon, dazomet, 2,4-DB, 3,4-DB, 2,4-DEB, desmedipham, dicamba, dichlobenil, ortho-dichlorobenzene, para-dichlorobenzene, dichlorprop, dichlorprop-P, diclofop, diclofop-methyl, diclosulam, difenzoquat, difenzoquat metilsulfate, diflufenican, diflufenzopyr, dimefuron, dimepiperate, dimethachlor, dimethametryn, dimethenamid, dimethenamid-P, dimethipin, dimethylarsinic acid, dinitramine, dinoterb, diphenamid, diquat, diquat dibromide, dithiopyr, diuron, DNOC, 3,4-DP, DSMA, EBEP, endothal, EPTC, esprocarb, ethalfluralin, ethametsulfuron, ethametsulfuron-methyl, ethofumesate, ethoxyfen, ethoxysulfuron, etobenzanid, fenoxaprop-P, fenoxaprop-P-ethyl, fentrazamide, ferrous sulfate, flamprop-M, flazasulfuron, florasulam, fluazifop, fluazifop-butyl, fluazifop-P, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, fluoroglycofen, fluoroglycofen-ethyl, flupropanate, flupyrsulfuron, flupyrsulfuron-methyl-sodium, flurenol, fluridone, fluorochloridone, fluoroxypyr, flurtamone, fluthiacet, fluthiacet-methyl, fomesafen, foramsulfuron, fosamine, glufosinate, glufosinate-ammonium, glyphosate, halosulfuron, halosulfuron-methyl, haloxyfop, haloxyfop-P, HC-252, hexazinone, imazamethabenz, imazamethabenz-methyl, imazamox, imazapic, imazapyr, imazaquin, imazethapyr, imazosulfuron, indanofan, iodomethane, iodosulfuron, iodosulfuron-methyl-sodium, ioxynil, isoproturon, isouron, isoxaben, isoxachlortole, isoxaflutole, karbutilate, lactofen, lenacil, linuron, MAA, MAMA, MCPA, MCPA-thioethyl, MCPB, mecoprop, mecoprop-P, mefenacet, mefluidide, mesosulfuron, mesosulfuron-methyl, mesotrione, metam, metamifop, metamitron, metazachlor, methabenzthiazuron, methylarsonic acid, methyldymron, methyl isothiocyanate, metobenzuron, metolachlor, S-metolachlor, metosulam, metoxuron, metribuzin, metsulfuron, metsulfuron-methyl, MK-66, molinate, monolinuron, MSMA, naproanilide, napropamide, naptalam, neburon, nicosulfuron, nonanoic acid, norflurazon, oleic acid (fatty acids), orbencarb, orthosulfamuron, oryzalin, oxadiargyl, oxadiazon, oxasulfuron, oxaziclomefone, oxyfluorfen, paraquat, paraquat dichloride, pebulate, pendimethalin, penoxsulam, pentachlorophenol, pentanochlor, pentoxazone, pethoxamid, petrolium oils, phenmedipham, phenmedipham-ethyl, picloram, picolinafen, pinoxaden, piperophos, potassium arsenite, potassium azide, pretilachlor, primisulfuron, primisulfuron-methyl, prodiamine, profluazol, profoxydim, prometon, prometryn, propachlor, propanil, propaquizafop, propazine, propham, propisochlor, propoxycarbazone, propoxycarbazone-sodium, propyzamide, prosulfocarb, prosulfuron, pyraclonil, pyraflufen, pyraflufen-ethyl, pyrazolynate, pyrazosulfuron, pyrazosulfuron-ethyl, pyrazoxyfen, pyribenzoxim, pyributicarb, pyridafol, pyridate, pyriftalid, pyriminobac, pyriminobac-methyl, pyrimisulfan, pyrithiobac, pyrithiobac-sodium, quinclorac, quinmerac, quinoclamine, quizalofop, quizalofop-P, rimsulfuron, sethoxydim, siduron, simazine, simetryn, SMA, sodium arsenite, sodium azide, sodium chlorate, sulcotrione, sulfentrazone, sulfometuron, sulfometuron-methyl, sulfosate, sulfosulfuron, sulfuric acid, tar oils, 2,3,6-TBA, TCA, TCA-sodium, tebuthiuron, tepraloxydim, terbacil, terbumeton, terbuthylazine, terbutryn, thenylchlor, thiazopyr, thifensulfuron, thifensulfuron-methyl, thiobencarb, tiocarbazil, topramezone, tralkoxydim, tri-allate, triasulfuron, triaziflam, tribenuron, tribenuron-methyl, tricamba, triclopyr, trietazine, trifloxysulfuron, trifloxysulfuron-sodium, trifluralin, triflusulfuron, triflusulfuron-methyl, trihydroxytriazine, tritosulfuron, [3-[2-chloro-4-fluoro-5-(-methyl-6-trifluoromethyl-2,4-dioxo-,2,3,4-t- etrahydropyrimidin-3-yl)phenoxy]-2-pyridyloxy]acetic acid ethyl ester (CAS RN 353292-3-6), 4-[(4,5-dihydro-3-methoxy-4-methyl-5-oxo)-H-,2,4-triazol--ylcarbonyl-sulfamoyl]-5-methylthiophene-3-carboxylic acid (BAY636), BAY747 (CAS RN 33504-84-2), topramezone (CAS RN 2063-68-8), 4-hydroxy-3-[[2-[(2-methoxyethoxy)methyl]-6-(trifluoro-methyl)-3-pyridi- nyl]carbonyl]-bicyclo[3.2.]oct-3-en-2-one (CAS RN 35200-68-5), and 4-hydroxy-3-[[2-(3-methoxypropyl)-6-(difluoromethyl)-3-pyridinyl]carbon-yl]-bicyclo[3.2.]oct-3-en-2-one. Additionally, including herbicidal compounds of unspecified modes of action as described in CN101279950A, CN101279951A, DE10000600A1, DE10116399A1, DE102004054666A1, DE102005014638A1, DE102005014906A1, DE102007012168A1, DE102010042866A1, DE10204951A1, DE10234875A1, DE10234876A1, DE10256353A1, DE10256354A1, DE10256367A1, EP1157991A2, EP1238586A1, EP2147919A1, EP2160098A2, JP03968012B2, JP2001253874A, JP2002080454A, JP2002138075A, JP2002145707A, JP2002220389A, JP2003064059A, JP2003096059A, JP2004051628A, JP2004107228A, JP2005008583A, JP2005239675A, JP2005314407A, JP2006232824A, JP2006282552A, JP2007153847A, JP2007161701A, JP2007182404A, JP2008074840A, JP2008074841A, JP2008133207A, JP2008133218A, JP2008169121A, JP2009067739A, JP2009114128A, JP2009126792A, JP2009137851A, US20060111241A1, US20090036311A1, US20090054240A1, US20090215628A1, US20100099561A1, US20100152443A1, US20110105329A1, US20110201501A1, WO2001055066A2, WO2001056975A1, WO2001056979A1, WO2001090071A2, WO2001090080A1, WO2002002540A1 , WO2002028182A1, WO2002040473A1, WO2002044173A2, WO2003000679A2, WO2003006422A1 , WO2003013247A1, WO2003016308A1, WO2003020704A1, WO2003022051A1, WO2003022831A1, WO2003022843A1, WO2003029243A2, WO2003037085A1, WO2003037878A1, WO2003045878A2, WO2003050087A2, WO2003051823A1, WO2003051824A1, WO2003051846A2, WO2003076409A1, WO2003087067A1, WO2003090539A1, WO2003091217A1, WO2003093269A2, WO2003104206A2, WO2004002947A1, WO2004002981A2, WO2004011429A1, WO2004029060A1, WO2004035545A2, WO2004035563A1, WO2004035564A1, WO2004037787A1, WO2004067518A1, WO2004067527A1, WO2004077950A1, WO2005000824A1, WO2005007627A1, WO2005040152A1, WO2005047233A1, WO2005047281A1, WO2005061443A2, WO2005061464A1, WO2005068434A1, WO2005070889A1, WO2005089551A1, WO2005095335A1, WO2006006569A1, WO2006024820A1, WO2006029828A1, WO2006029829A1, WO2006037945A1, WO2006050803A1, WO2006090792A1, WO2006123088A2, WO2006125687A1, WO2006125688A1, WO2007003294A1, WO2007026834A1, WO2007071900A1, WO2007077201A1, WO2007077247A1, WO2007096576A1, WO2007119434A1, WO2007134984A1, WO2008009908A1, WO2008029084A1, WO2008059948A1, WO2008071918A1, WO2008074991A1, WO2008084073A1, WO2008100426A2, WO2008102908A1, WO2008152072A2, WO2008152073A2, WO2009000757A1, WO2009005297A2, WO2009035150A2, WO2009063180A1, WO2009068170A2, WO2009068171A2, WO2009086041A1, WO2009090401A2, WO2009090402A2, WO2009115788A1, WO2009116558A1, WO2009152995A1, WO2009158258A1, WO2010012649A1, WO2010012649A1, WO2010026989A1, WO2010034153A1, WO2010049270A1, WO2010049369A1, WO2010049405A1, WO2010049414A1, WO2010063422A1, WO2010069802A1, WO2010078906A2, WO2010078912A1, WO2010104217A1, WO2010108611A1, WO2010112826A3, WO2010116122A3, WO2010119906A1, WO2010130970A1, WO2011003776A2, WO2011035874A1, WO2011065451A

Auxin-like herbicides include benzoic acid herbicide, phenoxy carboxylic acid herbicide, pyridine carboxylic acid herbicide, quinoline carboxylic acid herbicide, pyrimidine carboxylic acid herbicide, and benazolin-ethyl herbicide.

The benzoic acid herbicide group (dicamba (3,6-dichloro-o-anisic acid), chloramben (3-amino-2,5-dichlorobenzoic acid), and TBA (2,3,6-trichlorobenzoic acid)) are effective herbicides for both pre-emergence and post-emergence weed management. Dicamba is one of the many auxin-like herbicides that is a low-cost, environmentally friendly herbicide that has been used as a pre-emergence and post-emergence herbicide to effectively control annual and perennial broadleaf weeds and several grassy weeds in corn, sorghum, small grains, pasture, hay, rangeland, sugarcane, asparagus, turf, and grass seed crops (Crop Protection Chemicals Reference, pp. 1803-1821, Chemical & Pharmaceutical Press, Inc., New York, NY, 11th ed., 1995). Dicamba refers to 3,6-dichloro-o-anisic acid or 3,6-dichloro-2-methoxy benzoic acid and its acids and salts. Its salts include isopropylamine, diglycoamine, dimethylamine, potassium and sodium. Dicamba includes for example, commercial formulations without limitation, Banvel™ (as DMA salt, BASF, Research Triangle Park, NC), Clarity® (DGA salt, BASF), VEL-58-CS-11™ (BASF) and Vanquish™ (DGA salt, BASF). Dicamba is a useful herbicide as a tank mix, concomitantly, or pre or post treatment with the compositions.

An auxin-like herbicide also includes a phenoxy carboxylic acid compound, a pyridine carboxylic acid compound, a quinoline carboxylic acid compound, and a benazolin-ethyl compound. Examples of a phenoxy carboxylic acid compound include, but are not limited to 2,4-dichlorophenoxyacetic acid, (4-chloro-2-methylphenoxy) acetic acid, diclorprop (2,4-DP), mecoprop (MCPP), and clomeprop. Examples of pyridine herbicides include, but are not limited to clopryalid, picloram, fluroxypyr, aminocyclopyrachlor and triclopyr. These auxin-like herbicides are useful in a tank mix, concomitantly, or pre or post treatment with the compositions. Auxin-like herbicides include commercially available formulations, for example,including but not limited to 2,4-D, 2,4-DB (Butyrac® 200, Bakker), MCPA (Rhonox®, Rhomene), mecoprop, dichlorprop, 2,4,5-T, triclopyr (Garlon®, Dow AgroSciences, Indianapolis, IN), chloramben, dicamba (Banvel®, Clarity®, Oracle®, Sterling®), 2,3,6-TBA, tricamba, clopyralid (Stinger®, Dow AgroSciences), picloram (Tordon®, Dow AgroSciences), quinmerac, quinclorac, benazolin, fenac, IAA, NAA, orthonil and fluroxypyr (Vista®, Starane®, Dow AgroSciences), aminopyralid (Milestone®, Dow AgroSciences) and aminocyclopyrachlor (Dupont, Wilmington, DE).

The trigger polynucleotide and oligonucleotide molecule compositions are useful in compositions, such as liquids that comprise the polynucleotide molecules at low concentrations, alone or in combination with other components, for example one or more herbicide molecules, either in the same solution or in separately applied liquids that also provide a transfer agent. While there is no upper limit on the concentrations and dosages of polynucleotide molecules that can useful in the methods, lower effective concentrations and dosages will generally be sought for efficiency. The concentrations can be adjusted in consideration of the volume of spray or treatment applied to plant leaves or other plant part surfaces, such as flower petals, stems, tubers, fruit, anthers, pollen, or seed. In one embodiment, a useful treatment for herbaceous plants using 25-mer oligonucleotide molecules is about 1 nanomole (nmol) of oligonucleotide molecules per plant, for example, from about 0.05 to 1 nmol per plant. Other embodiments for herbaceous plants include useful ranges of about 0.05 to about 100 nmol, or about 0.1 to about 20 nmol, or about 1 nmol to about 10 nmol of polynucleotides per plant. Very large plants, trees, or vines may require correspondingly larger amounts of polynucleotides. When using long dsRNA molecules that can be processed into multiple oligonucleotides, lower concentrations can be used. To illustrate embodiments, the factor 1X, when applied to oligonucleotide molecules is arbitrarily used to denote a treatment of 0.8 nmol of polynucleotide molecule per plant; 10X, 8 nmol of polynucleotide molecule per plant; and 100X, 80 nmol of polynucleotide molecule per plant.

The polynucleotide compositions are useful in compositions, such as liquids that comprise polynucleotide molecules, alone or in combination with other components either in the same liquid or in separately applied liquids that provide a transfer agent. As used herein, a transfer agent is an agent that, when combined with a polynucleotide in a composition that is topically applied to a target plant surface, enables the polynucleotide to enter a plant cell. In certain embodiments, a transfer agent is an agent that conditions the surface of plant tissue, e.g., leaves, stems, roots, flowers, or fruits, to permeation by the polynucleotide molecules into plant cells. The transfer of polynucleotides into plant cells can be facilitated by the prior or contemporaneous application of a polynucleotide-transferring agent to the plant tissue. In some embodiments the transferring agent is applied subsequent to the application of the polynucleotide composition. The polynucleotide transfer agent enables a pathway for polynucleotides through cuticle wax barriers, stomata and/or cell wall or membrane barriers into plant cells. Suitable transfer agents to facilitate transfer of the polynucleotide into a plant cell include agents that increase permeability of the exterior of the plant or that increase permeability of plant cells to oligonucleotides or polynucleotides. Such agents to facilitate transfer of the composition into a plant cell include a chemical agent, or a physical agent, or combinations thereof. Chemical agents for conditioning or transfer include (a) surfactants, (b) an organic solvent or an aqueous solution or aqueous mixtures of organic solvents, (c) oxidizing agents, (d) acids, (e) bases, (f) oils, (g) enzymes, or combinations thereof. Embodiments of the method can optionally include an incubation step, a neutralization step (e.g., to neutralize an acid, base, or oxidizing agent, or to inactivate an enzyme), a rinsing step, or combinations thereof. Embodiments of agents or treatments for conditioning of a plant to permeation by polynucleotides include emulsions, reverse emulsions, liposomes, and other micellar-like compositions. Embodiments of agents or treatments for conditioning of a plant to permeation by polynucleotides include counter-ions or other molecules that are known to associate with nucleic acid molecules, *e.g.,* inorganic ammonium ions, alkyl ammonium ions, lithium ions, polyamines such as spermine, spermidine, or putrescine, and other cations. Organic solvents useful in conditioning a plant to permeation by polynucleotides include DMSO, DMF, pyridine, *N*-pyrrolidine, hexamethylphosphoramide, acetonitrile, dioxane, polypropylene glycol, other solvents miscible with water or that will dissolve phosphonucleotides in non-aqueous systems (such as is used in synthetic reactions). Naturally derived or synthetic oils with or without surfactants or emulsifiers can be used, *e. g.,* plant-sourced oils, crop oils (such as those listed in the 9^{th} Compendium of Herbicide Adjuvants, publicly available on the worldwide web (internet) at herbicide.adjuvants.com can be used, *e.g.,* paraffinic oils, polyol fatty acid esters, or oils with short-chain molecules modified with amides or polyamines such as polyethyleneimine or *N*-pyrrolidine. Transfer agents include, but are not limited to, organosilicone preparations.

An agronomic field in need of plant control is treated by application of an agricultural chemical composition directly to the surface of the growing plants, such as by a spray. For example, the method is applied to control weeds in a field of crop plants by spraying the field with the composition. The composition can be provided as a tank mix with one or more herbicidal chemical and additional pesticidal chemicals to control pests and diseases of the crop plants in need of pest and disease control, a sequential treatment of components (generally the polynucleotide containing composition followed by the herbicide), or a simultaneous treatment or mixing of one or more of the components of the composition from separate containers. Treatment of the field can occur as often as needed to provide weed control and the components of the composition can be adjusted to target specific weed species or weed families through utilization of specific polynucleotides or polynucleotide compositions capable of selectively targeting the specific species or plant family to be controlled. The composition can be applied at effective use rates according to the time of application to the field, for example, preplant, at planting, post planting, post harvest. Glyphosate can be applied to a field at rates of 11-44 ounces/acre upto 7.2875 pounds/acre. The polynucleotides of the composition can be applied at rates of 1 to 30 grams per acre depending on the number of trigger molecules needed for the scope of weeds in the field.

Crop plants in which weed control may be needed include but are not limited to corn, soybean, cotton, canola, sugar beet, alfalfa, sugarcane, rice, and wheat; vegetable plants including, but not limited to, tomato, sweet pepper, hot pepper, melon, watermelon, cucumber, eggplant, cauliflower, broccoli, lettuce, spinach, onion, peas, carrots, sweet corn, Chinese cabbage, leek, fennel, pumpkin, squash or gourd, radish, Brussels sprouts, tomatillo, garden beans, dry beans, or okra; culinary plants including, but not limited to, basil, parsley, coffee, or tea; or fruit plants including but not limited to apple, pear, cherry, peach, plum, apricot, banana, plantain, table grape, wine grape, citrus, avocado, mango, or berry; a tree grown for ornamental or commercial use, including, but not limited to, a fruit or nut tree; ornamental plant (e.g., an ornamental flowering plant or shrub or turf grass). The methods and compositions provided herein can also be applied to plants produced by a cutting, cloning, or grafting process (i.e., a plant not grown from a seed) including fruit trees and plants that include, but are not limited to, avocados, tomatoes, eggplant, cucumber, melons, watermelons, and grapes as well as various ornamental plants.

### Pesticidal Mixtures

The polynucleotide compositions may also be used as mixtures with various agricultural chemicals and/or insecticides, miticides and fungicides, pesticidal and biopesticidal agents. Examples include but are not limited to azinphos-methyl, acephate, isoxathion, isofenphos, ethion, etrimfos, oxydemeton-methyl, oxydeprofos, quinalphos, chlorpyrifos, chlorpyrifos-methyl, chlorfenvinphos, cyanophos, dioxabenzofos, dichlorvos, disulfoton, dimethylvinphos, dimethoate, sulprofos, diazinon, thiometon, tetrachlorvinphos, temephos, tebupirimfos, terbufos, naled, vamidothion, pyraclofos, pyridafenthion, pirimiphos-methyl, fenitrothion, fenthion, phenthoate, flupyrazophos, prothiofos, propaphos, profenofos, phoxime, phosalone, phosmet, formothion, phorate, malathion, mecarbam, mesulfenfos, methamidophos, methidathion, parathion, methyl parathion, monocrotophos, trichlorphon, EPN, isazophos, isamidofos, cadusafos, diamidaphos, dichlofenthion, thionazin, fenamiphos, fosthiazate, fosthietan, phosphocarb, DSP, ethoprophos, alanycarb, aldicarb, isoprocarb, ethiofencarb, carbaryl, carbosulfan, xylylcarb, thiodicarb, pirimicarb, fenobucarb, furathiocarb, propoxur, bendiocarb, benfuracarb, methomyl, metolcarb, XMC, carbofuran, aldoxycarb, oxamyl, acrinathrin, allethrin, esfenvalerate, empenthrin, cycloprothrin, cyhalothrin, gamma-cyhalothrin, lambda-cyhalothrin, cyfluthrin, beta-cyfluthrin, cypermethrin, alpha-cypermethrin, zeta-cypermethrin, silafluofen, tetramethrin, tefluthrin, deltamethrin, tralomethrin, bifenthrin, phenothrin, fenvalerate, fenpropathrin, furamethrin, prallethrin, flucythrinate, fluvalinate, flubrocythrinate, permethrin, resmethrin, ethofenprox, cartap, thiocyclam, bensultap, acetamiprid, imidacloprid, clothianidin, dinotefuran, thiacloprid, thiamethoxam, nitenpyram, chlorfluazuron, diflubenzuron, teflubenzuron, triflumuron, novaluron, noviflumuron, bistrifluoron, fluazuron, flucycloxuron, flufenoxuron, hexaflumuron, lufenuron, chromafenozide, tebufenozide, halofenozide, methoxyfenozide, diofenolan, cyromazine, pyriproxyfen, buprofezin, methoprene, hydroprene, kinoprene, triazamate, endosulfan, chlorfenson, chlorobenzilate, dicofol, bromopropylate, acetoprole, fipronil, ethiprole, pyrethrin, rotenone, nicotine sulphate, BT (Bacillus Thuringiensis) agent, spinosad, abamectin, acequinocyl, amidoflumet, amitraz, etoxazole, chinomethionat, clofentezine, fenbutatin oxide, dienochlor, cyhexatin, spirodiclofen, spiromesifen, tetradifon, tebufenpyrad, binapacryl, bifenazate, pyridaben, pyrimidifen, fenazaquin, fenothiocarb, fenpyroximate, fluacrypyrim, fluazinam, flufenzin, hexythiazox, propargite, benzomate, polynactin complex, milbemectin, lufenuron, mecarbam, methiocarb, mevinphos, halfenprox, azadirachtin, diafenthiuron, indoxacarb, emamectin benzoate, potassium oleate, sodium oleate, chlorfenapyr, tolfenpyrad, pymetrozine, fenoxycarb, hydramethylnon, hydroxy propyl starch, pyridalyl, flufenerim, flubendiamide, flonicamid, metaflumizole, lepimectin, TPIC, albendazole, oxibendazole, oxfendazole, trichlamide, fensulfothion, fenbendazole, levamisole hydrochloride, morantel tartrate, dazomet, metam-sodium, triadimefon, hexaconazole, propiconazole, ipconazole, prochloraz, triflumizole, tebuconazole, epoxiconazole, difenoconazole, flusilazole, triadimenol, cyproconazole, metconazole, fluquinconazole, bitertanol, tetraconazole, triticonazole, flutriafol, penconazole, diniconazole, fenbuconazole, bromuconazole, imibenconazole, simeconazole, myclobutanil, hymexazole, imazalil, furametpyr, thifluzamide, etridiazole, oxpoconazole, oxpoconazole fumarate, pefurazoate, prothioconazole, pyrifenox, fenarimol, nuarimol, bupirimate, mepanipyrim, cyprodinil, pyrimethanil, metalaxyl, mefenoxam, oxadixyl, benalaxyl, thiophanate, thiophanate-methyl, benomyl, carbendazim, fuberidazole, thiabendazole, manzeb, propineb, zineb, metiram, maneb, ziram, thiuram, chlorothalonil, ethaboxam, oxycarboxin, carboxin, flutolanil, silthiofam, mepronil, dimethomorph, fenpropidin, fenpropimorph, spiroxamine, tridemorph, dodemorph, flumorph, azoxystrobin, kresoxim-methyl, metominostrobin, orysastrobin, fluoxastrobin, trifloxystrobin, dimoxystrobin, pyraclostrobin, picoxystrobin, iprodione, procymidone, vinclozolin, chlozolinate, flusulfamide, dazomet, methyl isothiocyanate, chloropicrin, methasulfocarb, hydroxyisoxazole, potassium hydroxyisoxazole, echlomezol, D-D, carbam, basic copper chloride, basic copper sulfate, copper nonylphenolsulfonate, oxine copper, DBEDC, anhydrous copper sulfate, copper sulfate pentahydrate, cupric hydroxide, inorganic sulfur, wettable sulfur, lime sulfur, zinc sulfate, fentin, sodium hydrogen carbonate, potassium hydrogen carbonate, sodium hypochlorite, silver, edifenphos, tolclofos-methyl, fosetyl, iprobenfos, dinocap, pyrazophos, carpropamid, fthalide, tricyclazole, pyroquilon, diclocymet, fenoxanil, kasugamycin, validamycin, polyoxins, blasticiden S, oxytetracycline, mildiomycin, streptomycin, rape seed oil, machine oil, benthiavalicarbisopropyl, iprovalicarb, propamocarb, diethofencarb, fluoroimide, fludioxanil, fenpiclonil, quinoxyfen, oxolinic acid, chlorothalonil, captan, folpet, probenazole, acibenzolar-S-methyl, tiadinil, cyflufenamid, fenhexamid, diflumetorim, metrafenone, picobenzamide, proquinazid, famoxadone, cyazofamid, fenamidone, zoxamide, boscalid, cymoxanil, dithianon, fluazinam, dichlofluanide, triforine, isoprothiolane, ferimzone, diclomezine, tecloftalam, pencycuron, chinomethionat, iminoctadine acetate, iminoctadine albesilate, ambam, polycarbamate, thiadiazine, chloroneb, nickel dimethyldithiocarbamate, guazatine, dodecylguanidine-acetate, quintozene, tolylfluanid, anilazine, nitrothalisopropyl, fenitropan, dimethirimol, benthiazole, harpin protein, flumetover, mandipropamide and penthiopyrad.

### Polynucleotides

As used herein, the term "DNA", "DNA molecule", "DNA polynucleotide molecule" refers to a single-stranded DNA (ssDNA) or double-stranded DNA (dsDNA) molecule of genomic or synthetic origin, such as, a polymer of deoxyribonucleotide bases or a DNA polynucleotide molecule. As used herein, the term "DNA sequence", "DNA nucleotide sequence" or "DNA polynucleotide sequence" refers to the nucleotide sequence of a DNA molecule. As used herein, the term "RNA", "RNA molecule", "RNA polynucleotide molecule" refers to a single-stranded RNA (ssRNA) or double-stranded RNA (dsRNA) molecule of genomic or synthetic origin, such as, a polymer of ribonucleotide bases that comprise single or double stranded regions. Unless otherwise stated, nucleotide sequences in the text of this specification are given, when read from left to right, in the 5' to 3' direction. The nomenclature used herein is that required by Title 37 of the United States Code of Federal Regulations § 1.822 and set forth in the tables in WIPO Standard ST.25 (1998), Appendix 2, Tables 1 and 3.

As used herein, "polynucleotide" refers to a DNA or RNA molecule containing multiple nucleotides and generally refers both to "oligonucleotides" (a polynucleotide molecule of typically 50 or fewer nucleotides in length) and polynucleotides of 51 or more nucleotides. Embodiments include compositions including oligonucleotides having a length of 18-25 nucleotides (18-mers, 19-mers, 20-mers, 21-mers, 22-mers, 23-mers, 24-mers, or 25-mers), for example, oligonucleotides SEQ ID NO:3223-3542 or fragments thereof or medium-length polynucleotides having a length of 26 or more nucleotides (polynucleotides of 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, about 65, about 70, about 75, about 80, about 85, about 90, about 95, about 100, about 110, about 120, about 130, about 140, about 150, about 160, about 170, about 180, about 190, about 200, about 210, about 220, about 230, about 240, about 250, about 260, about 270, about 280, about 290, or about 300 nucleotides), for example, oligonucleotides SEQ ID NO:121-3222 or fragments thereof or long polynucleotides having a length greater than about 300 nucleotides (for example, polynucleotides of between about 300 to about 400 nucleotides, between about 400 to about 500 nucleotides, between about 500 to about 600 nucleotides, between about 600 to about 700 nucleotides, between about 700 to about 800 nucleotides, between about 800 to about 900 nucleotides, between about 900 to about 1000 nucleotides, between about 300 to about 500 nucleotides, between about 300 to about 600 nucleotides, between about 300 to about 700 nucleotides, between about 300 to about 800 nucleotides, between about 300 to about 900 nucleotides, or about 1000 nucleotides in length, or even greater than about 1000 nucleotides in length, for example up to the entire length of a target gene including coding or non-coding or both coding and non-coding portions of the target gene), for example, polynucleotides of Table 1 (SEQ ID NO:1-120), wherein the selected polynucleotides or fragments thereof are homologous or complementary to SEQ ID NO:1-120 and suppresses, represses or otherwise delay the expression of the target EPSPS gene. Where a polynucleotide is double-stranded, its length can be similarly described in terms of base pairs. A target gene comprises any polynucleotide molecule in a plant cell or fragment thereof for which the modulation of the expression of the target gene is provided by the methods and compositions. A gene has noncoding genetic elements (components) that provide for the function of the gene, these elements are polynucleotides that provide gene expression regulation, such as, a promoter, an enhancer, a 5' untranslated region, intron regions, and a 3' untranslated region. Oligonucleotides and polynucleotides can be made to any of the genetic elements of a gene and to polynucleotides spanning the junction region of a genetic element, such as, an intron and exon, the junction region of a promoter and a transcribed region, the junction region of a 5' leader and a coding sequence, the junction of a 3' untranslated region and a coding sequence.

Polynucleotide compositions used in the various embodiments include compositions including oligonucleotides or polynucleotides or a mixture of both, including RNA or DNA or RNA/DNA hybrids or chemically modified oligonucleotides or polynucleotides or a mixture thereof. In some embodiments, the polynucleotide may be a combination of ribonucleotides and deoxyribonucleotides, for example, synthetic polynucleotides consisting mainly of ribonucleotides but with one or more terminal deoxyribonucleotides or synthetic polynucleotides consisting mainly of deoxyribonucleotides but with one or more terminal dideoxyribonucleotides. In some embodiments, the polynucleotide includes non-canonical nucleotides such as inosine, thiouridine, or pseudouridine. In some embodiments, the polynucleotide includes chemically modified nucleotides. Examples of chemically modified oligonucleotides or polynucleotides are well known in the art; see, for example, US Patent Publication 20110171287, US Patent Publication 20110171176, and US Patent Publication 20110152353, US Patent Publication, 20110152346, US Patent Publication 20110160082 . For example, including but not limited to the naturally occurring phosphodiester backbone of an oligonucleotide or polynucleotide can be partially or completely modified with phosphorothioate, phosphorodithioate, or methylphosphonate internucleotide linkage modifications, modified nucleoside bases or modified sugars can be used in oligonucleotide or polynucleotide synthesis, and oligonucleotides or polynucleotides can be labeled with a fluorescent moiety (for example, fluorescein or rhodamine) or other label (for example, biotin).

The polynucleotides can be single- or double-stranded RNA or single- or double-stranded DNA or double-stranded DNA/RNA hybrids or modified analogues thereof, and can be of oligonucleotide lengths or longer. In more specific embodiments, the polynucleotides that provide single-stranded RNA in the plant cell are selected from the group consisting of (a) a single-stranded RNA molecule (ssRNA), (b) a single-stranded RNA molecule that self-hybridizes to form a double-stranded RNA molecule, (c) a double-stranded RNA molecule (dsRNA), (d) a single-stranded DNA molecule (ssDNA), (e) a single-stranded DNA molecule that self-hybridizes to form a double-stranded DNA molecule, and (f) a single-stranded DNA molecule including a modified Pol III gene that is transcribed to an RNA molecule, (g) a double-stranded DNA molecule (dsDNA), (h) a double-stranded DNA molecule including a modified Pol III gene that is transcribed to an RNA molecule, (i) a double-stranded, hybridized RNA/DNA molecule, or combinations thereof. In some embodiments these polynucleotides include chemically modified nucleotides or non-canonical nucleotides. In some embodiments, the oligonucleotides may be blunt-ended or may comprise a 3' overhang of from 1-5 nucleotides of at least one or both of the strands. Other configurations of the oligonucleotide are known in the field and are contemplated herein. In embodiments of the method the polynucleotides include double-stranded DNA formed by intramolecular hybridization, double-stranded DNA formed by intermolecular hybridization, double-stranded RNA formed by intramolecular hybridization, or double-stranded RNA formed by intermolecular hybridization. In one embodiment the polynucleotides include single-stranded DNA or single-stranded RNA that self-hybridizes to form a hairpin structure having an at least partially double-stranded structure including at least one segment that will hybridize to RNA transcribed from the gene targeted for suppression. Not intending to be bound by any mechanism, it is believed that such polynucleotides are or will produce single-stranded RNA with at least one segment that will hybridize to RNA transcribed from the gene targeted for suppression. In certain other embodiments the polynucleotides further includes a promoter, generally a promoter functional in a plant, for example, a pol II promoter, a pol III promoter, a pol IV promoter, or a pol V promoter.

The term "gene" refers to components that comprise chromosomal DNA, plasmid DNA, cDNA, intron and exon DNA, artificial DNA polynucleotide, or other DNA that encodes a peptide, polypeptide, protein, or RNA transcript molecule, and the genetic elements flanking the coding sequence that are involved in the regulation of expression, such as, promoter regions, 5' leader regions, 3' untranslated region that may exist as native genes or transgenes in a plant genome. The gene or a fragment thereof is isolated and subjected to polynucleotide sequencing methods that determines the order of the nucleotides that comprise the gene. Any of the components of the gene are potential targets for a trigger oligonucleotide and polynucleotides.

The trigger polynucleotide molecules are designed to modulate expression by inducing regulation or suppression of an endogenous EPSPS gene in a plant and are designed to have a nucleotide sequence essentially identical or essentially complementary to the nucleotide sequence of an endogenous EPSPS gene of a plant or to the sequence of RNA transcribed from an endogenous EPSPS gene of a plant, the sequence thereof determined by isolating the gene or a fragment of the gene from the plant, which can be coding sequence or non-coding sequence. Effective molecules that modulate expression are referred to as "a trigger molecule, or trigger polynucleotide". By "essentially identical" or "essentially complementary" is meant that the trigger polynucleotides (or at least one strand of a double-stranded polynucleotide or portion thereof, or a portion of a single strand polynucleotide) are designed to hybridize to the endogenous gene noncoding sequence or to RNA transcribed (known as messenger RNA or an RNA transcript) from the endogenous gene to effect regulation or suppression of expression of the endogenous gene. Trigger molecules are identified by "tiling" the gene targets with partially overlapping probes or non-overlapping probes of antisense or sense polynucleotides that are essentially identical or essentially complementary to the nucleotide sequence of an endogenous gene. Multiple target sequences can be aligned and sequence regions with homology in common, according to the methods, are identified as potential trigger molecules for the multiple targets. Multiple trigger molecules of various lengths, for example 18-25 nucleotides, 26-50 nucleotides, 51-100 nucleotides, 101-200 nucleotides, 201-300 nucleotides or more can be pooled into a few treatments in order to investigate polynucleotide molecules that cover a portion of a gene sequence (for example, a portion of a coding versus a portion of a noncoding region, or a 5' versus a 3' portion of a gene) or an entire gene sequence including coding and noncoding regions of a target gene. Polynucleotide molecules of the pooled trigger molecules can be divided into smaller pools or single molecules inorder to identify trigger molecules that provide the desired effect.

The target gene RNA and DNA polynucleotide molecules (Table 1, SEQ ID NO:1-120) are sequenced by any number of available methods and equipment. Some of the sequencing technologies are available commercially, such as the sequencing-by-hybridization platform from Affymetrix Inc. (Sunnyvale, Calif.) and the sequencing-by-synthesis platforms from 454 Life Sciences (Bradford, Conn.), Illumina/Solexa (Hayward, Calif.) and Helicos Biosciences (Cambridge, Mass.), and the sequencing-by-ligation platform from Applied Biosystems (Foster City, Calif.), as described below. In addition to the single molecule sequencing performed using sequencing-by-synthesis of Helicos Biosciences, other single molecule sequencing technologies are encompassed and include the SMRT™. technology of Pacific Biosciences, the Ion Torrent™. technology, and nanopore sequencing being developed for example, by Oxford Nanopore Technologies. An EPSPS target gene comprising DNA or RNA can be isolated using primers or probes essentially complementary or essentially homologous to SEQ ID NO:1-120 or a fragment thereof. A polymerase chain reaction (PCR) gene fragment can be produced using primers essentially complementary or essentially homologous to SEQ ID NO:1-120 or a fragment thereof that is useful to isolate an EPSPS gene from a plant genome. SEQ ID NO: 1-120 or fragments thereof can be used in various sequence capture technologies to isolate additional target gene sequences, for example, including but not limited to Roche NimbleGen® (Madison, WI) and Streptavdin-coupled Dynabeads® (Life Technologies, Grand Island, NY) and US20110015084

Embodiments of functional single-stranded polynucleotides have sequence complementarity that need not be 100 percent, but is at least sufficient to permit hybridization to RNA transcribed from the target gene or DNA of the target gene to form a duplex to permit a gene silencing mechanism. Thus, in embodiments, a polynucleotide fragment is designed to be essentially identical to, or essentially complementary to, a sequence of 18 or more contiguous nucleotides in either the target EPSPS gene sequence or messenger RNA transcribed from the target gene. By "essentially identical" is meant having 100 percent sequence identity or at least about 83, 84, 85, 86, 87, 88, 89, 90, 91, 92, 93, 94, 95, 96, 97, 98, or 99 percent sequence identity when compared to the sequence of 18 or more contiguous nucleotides in either the target gene or RNA transcribed from the target gene; by "essentially complementary" is meant having 100 percent sequence complementarity or at least about 83, 84, 85, 86, 87, 88, 89, 90, 91, 92, 93, 94, 95, 96, 97, 98, or 99 percent sequence complementarity when compared to the sequence of 18 or more contiguous nucleotides in either the target gene or RNA transcribed from the target gene. In some embodiments, polynucleotide molecules are designed to have 100 percent sequence identity with or complementarity to one allele or one family member of a given target gene (coding or non-coding sequence of a gene); in other embodiments the polynucleotide molecules are designed to have 100 percent sequence identity with or complementarity to multiple alleles or family members of a given target gene.

"Identity" refers to the degree of similarity between two polynucleic acid or protein sequences. An alignment of the two sequences is performed by a suitable computer program. A widely used and accepted computer program for performing sequence alignments is CLUSTALW v1.6 (Thompson, et al. Nucl. Acids Res., 22: 4673-4680, 1994). The number of matching bases or amino acids is divided by the total number of bases or amino acids, and multiplied by 100 to obtain a percent identity. For example, if two 580 base pair sequences had 145 matched bases, they would be 25 percent identical. If the two compared sequences are of different lengths, the number of matches is divided by the shorter of the two lengths. For example, if there are 100 matched amino acids between a 200 and a 400 amino acid protein, they are 50 percent identical with respect to the shorter sequence. If the shorter sequence is less than 150 bases or 50 amino acids in length, the number of matches are divided by 150 (for nucleic acid bases) or 50 (for amino acids), and multiplied by 100 to obtain a percent identity.

Trigger molecules for specific gene family members can be identified from coding and/or non-coding sequences of gene families of a plant or multiple plants, by aligning and selecting 200-300 polynucleotide fragments from the least homologous regions amongst the aligned sequences and evaluated using topically applied polynucleotides (as sense or anti-sense ssDNA or ssRNA, dsRNA, or dsDNA) to determine their relative effectiveness in providing the herbicidal phenotype. The effective segments are further subdivided into 50-60 polynucleotide fragments, prioritized by least homology, and reevaluated using topically applied polynucleotides. The effective 50-60 polynucleotide fragments are subdivided into 19-30 polynucleotide fragments, prioritized by least homology, and again evaluated for induction of the herbicidal phenotype. Once relative effectiveness is determined, the fragments are utilized singly, or again evaluated in combination with one or more other fragments to determine the trigger composition or mixture of trigger polynucleotides for providing the herbicidal phenotype.

Trigger molecules for broad activity can be identified from coding and/or non-coding sequences of gene families of a plant or multiple plants, by aligning and selecting 200-300 polynucleotide fragments from the most homologous regions amongst the aligned sequences and evaluated using topically applied polynucleotides (as sense or anti-sense ssDNA or ssRNA, dsRNA, or dsDNA) to determine their relative effectiveness in inducing the herbicidal phenotype. The effective segments are subdivided into 50-60 polynucleotide fragments, prioritized by most homology, and reevaluated using topically applied polynucleotides. The effective 50-60 polynucleotide fragments are subdivided into 19-30 polynucleotide fragments, prioritized by most homology, and again evaluated for induction of the herbicidal phenotype. Once relative effectiveness is determined, the fragments may be utilized singly, or in combination with one or more other fragments to determine the trigger composition or mixture of trigger polynucleotides for providing the herbicidal phenotype.

Methods of making polynucleotides are well known in the art. Chemical synthesis, in vivo synthesis and in vitro synthesis methods and compositions are known in the art and include various viral elements, microbial cells, modified polymerases, and modified nucleotides. Commercial preparation of oligonucleotides often provides two deoxyribonucleotides on the 3' end of the sense strand. Long polynucleotide molecules can be synthesized from commercially available kits, for example, kits from Applied Biosystems/Ambion (Austin, TX) have DNA ligated on the 5' end in a microbial expression cassette that includes a bacterial T7 polymerase promoter that makes RNA strands that can be assembled into a dsRNA and kits provided by vaious manufacturers that include T7 RiboMax Express (Promega, Madison, WI), AmpliScribe T7-Flash (Epicentre, Madison, WI), and TranscriptAid T7 High Yield (Fermentas, Glen Burnie, MD). dsRNA molecules can be produced from microbial expression cassettes in bacterial cells (Ongvarrasopone et al. ScienceAsia 33:35-39; Yin, Appl. Microbiol. Biotechnol 84:323-333, 2009; Liu et al., BMC Biotechnology 10:85, 2010) that have regulated or deficient RNase III enzyme activity or the use of various viral vectors to produce sufficient quantities of dsRNA. EPSPS gene fragments are inserted into the microbial expression cassettes in a position in which the fragments are express to produce ssRNA or dsRNA useful in the methods described herein to regulate expression on a target EPSPS gene. Long polynucleotide molecules can also be assembled from multiple RNA or DNA fragments. In some embodiments design parameters such as Reynolds score (Reynolds et al. Nature Biotechnology 22, 326 - 330 (2004),Tuschl rules (Pei and Tuschl, Nature Methods 3(9): 670-676, 2006), i-score (Nucleic Acids Res 35: e123, 2007), i-Score Designer tool and associated algorithms (Nucleic Acids Res 32: 936-948, 2004. Biochem Biophys Res Commun 316: 1050-1058, 2004, Nucleic Acids Res 32: 893-901, 2004, Cell Cycle 3: 790-5, 2004, Nat Biotechnol 23: 995-1001, 2005, Nucleic Acids Res 35: e27, 2007, BMC Bioinformatics 7: 520, 2006, Nucleic Acids Res 35: e123, 2007, Nat Biotechnol 22: 326-330, 2004) are known in the art and may be used in selecting polynucleotide sequences effective in gene silencing. In some embodiments the sequence of a polynucleotide is screened against the genomic DNA of the intended plant to minimize unintentional silencing of other genes.

Ligands can be tethered to a polynucleotide, for example a dsRNA, ssRNA, dsDNA or ssDNA. Ligands in general can include modifiers, e.g., for enhancing uptake; diagnostic compounds or reporter groups e.g., for monitoring distribution; cross-linking agents; nuclease-resistance conferring moieties; and natural or unusual nucleobases. General examples include lipophiles, lipids (e.g., cholesterol, a bile acid, or a fatty acid (e.g., lithocholic-oleyl, lauroyl, docosnyl, stearoyl, palmitoyl, myristoyl oleoyl, linoleoyl), steroids (e.g., uvaol, hecigenin, diosgenin), terpenes (e.g., triterpenes, e.g., sarsasapogenin, Friedelin, epifriedelanol derivatized lithocholic acid), vitamins (e.g., folic acid, vitamin A, biotin, pyridoxal), carbohydrates, proteins, protein binding agents, integrin targeting molecules, polycationics, peptides, polyamines, and peptide mimics. The ligand may also be a recombinant or synthetic molecule, such as a synthetic polymer, e.g., polyethylene glycol (PEG), PEG-40K, PEG-20K and PEG-5K. Other examples of ligands include lipophilic molecules, e.g, cholesterol, cholic acid, adamantane acetic acid, 1-pyrene butyric acid, dihydrotestosterone, glycerol (e.g., esters and ethers thereof, e.g., C.sub.10, C.sub.11, C.sub.12, C.sub.13, C.sub.14, C.sub.15, C.sub.16, C.sub.17, C.sub.18, C.sub.19, or C.sub.20 alkyl; e.g., lauroyl, docosnyl, stearoyl, oleoyl, linoleoyl 1,3-bis-O(hexadecyl)glycerol, 1,3-bis-O(octaadecyl)glycerol), geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dodecanoyl, lithocholyl, 5.beta.-cholanyl, N,N-distearyl-lithocholamide, 1,2-di-O-stearoylglyceride, dimethoxytrityl, or phenoxazine) and PEG (e.g., PEG-5K, PEG-20K, PEG-40K). Preferred lipophilic moieties include lipid, cholesterols, oleyl, retinyl, or cholesteryl residues.

Conjugating a ligand to a dsRNA can enhance its cellular absorption, lipophilic compounds that have been conjugated to oligonucleotides include 1-pyrene butyric acid, 1,3-bis-O-(hexadecyl)glycerol, and menthol. One example of a ligand for receptor-mediated endocytosis is folic acid. Folic acid enters the cell by folate-receptor-radiated endocytosis. dsRNA compounds bearing folic acid would be efficiently transported into the cell via the folate-receptor-mediated endocytosis. Other ligands that have been conjugated to oligonucleotides include polyethylene glycols, carbohydrate clusters, cross-linking agents, porphyrin conjugates, delivery peptides and lipids such as cholesterol. In certain instances, conjugation of a cationic ligand to oligonucleotides results in improved resistance to nucleases. Representative examples of cationic ligands are propylammonium and dimethylpropylammonium. Interestingly, antisense oligonucleotides were reported to retain their high binding affinity to mRNA when the cationic ligand was dispersed, throughout the oligonucleotide. See M. Manoharan Antisense & Nucleic Acid Drug Development 2002, 12, 103 and references therein.

A biologic delivery can be accomplished by a variety of methods including, without limitation, (1) loading liposomes with a dsRNA acid molecule provided herein and (2) complexing a dsRNA molecule with lipids or liposomes to form nucleic acid-lipid or nucleic acid-liposome complexes. The liposome can be composed of cationic and neutral lipids commonly used to transfect cells in vitro. Cationic lipids can complex (e.g., charge-associate) with negatively charged, nucleic acids to form liposomes. Examples of cationic liposomes include, without limitation, lipofectin, lipofectamine, lipofectace, and DOTAP. Procedures for forming liposomes are well known in the art. Liposome compositions can be formed, for example, from phosphatidylcholine, dimyristoyl phosphatidylcholine, dipalmitoyl phosphatidylcholine, dimyristoyl phosphatidyl glycerol, dioleoyl phosphatidylethanolamine or liposomes comprising dihydrosphingomyelin (DHSM) Numerous lipophilic agents are commercially available, including Lipofectin® (Invitrogen/Life Technologies, Carlsbad, Calif.) and Effectene™ (Qiagen, Valencia, Calif.), In addition, systemic delivery methods can be optimized using commercially available cationic lipids such as DDAB or DOTAP, each of which can be mixed with a neutral lipid such as DOPE or cholesterol. In some eases, liposomes such as those described by Templeton et al. (Nature Biotechnology, 15:647-652 (1997)) can be used. In other embodiments, polycations such as polyethyleneimine can be used to achieve delivery in vivo and ex vivo (Boletta et al., J. Am Soc. Nephrol. 7:1728 (1996)). Additional information regarding the use of liposomes to deliver nucleic acids can be found in U.S. Pat. No. 6,271,359, PCT Publication WO 96/40964 and Morrissey, D. et al. 2005. Nat Biotechnol. 23(8):1002-7.

In certain embodiments, an organosilicone preparation that is commercially available as Silwet® L-77 surfactant having CAS Number 27306-78-1 and EPA Number: CAL.REG.NO. 5905-50073-AA, and currently available from Momentive Performance Materials, Albany, New York can be used to prepare a polynucleotide composition. In certain embodiments where a Silwet L-77 organosilicone preparation is used as a pre-spray treatment of plant leaves or other plant surfaces, freshly made concentrations in the range of about 0.015 to about 2 percent by weight (wt percent) (*e.g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) are efficacious in preparing a leaf or other plant surface for transfer of polynucleotide molecules into plant cells from a topical application on the surface. In certain embodiments of the methods and compositions provided herein, a composition that comprises a polynucleotide molecule and an organosilicone preparation comprising Silwet L-77 in the range of about 0.015 to about 2 percent by weight (wt percent) (*e.g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) is used or provided.

In certain embodiments, any of the commercially available organosilicone preparations provided such as the following Breakthru S 321, Breakthru S 200 Cat# 67674-67-3, Breakthru OE 441 Cat#68937-55-3, Breakthru S 278 Cat #27306-78-1, Breakthru S 243, Breakthru S 233 Cat#134180-76-0, available from manufacturer Evonik Goldschmidt (Germany), Silwet® HS 429, Silwet® HS 312, Silwet® HS 508, Silwet® HS 604 (Momentive Performance Materials, Albany, New York) can be used as transfer agents in a polynucleotide composition. In certain embodiments where an organosilicone preparation is used as a pre-spray treatment of plant leaves or other surfaces, freshly made concentrations in the range of about 0.015 to about 2 percent by weight (wt percent) (*e.g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) are efficacious in preparing a leaf or other plant surface for transfer of polynucleotide molecules into plant cells from a topical application on the surface. In certain embodiments of the methods and compositions provided herein, a composition that comprises a polynucleotide molecule and an organosilicone preparation in the range of about 0.015 to about 2 percent by weight (wt percent) (*e.g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) is used or provided.

Organosilicone preparations used in the methods and compositions provided herein can comprise one or more effective organosilicone compounds. As used herein, the phrase "effective organosilicone compound" is used to describe any organosilicone compound that is found in an organosilicone preparation that enables a polynucleotide to enter a plant cell. In certain embodiments, an effective organosilicone compound can enable a polynucleotide to enter a plant cell in a manner permitting a polynucleotide mediated suppression of a target gene expression in the plant cell. In general, effective organosilicone compounds include, but are not limited to, compounds that can comprise: i) a trisiloxane head group that is covalently linked to, ii) an alkyl linker including, but not limited to, an n-propyl linker, that is covalently linked to, iii) a poly glycol chain, that is covalently linked to, iv) a terminal group. Trisiloxane head groups of such effective organosilicone compounds include, but are not limited to, heptamethyltrisiloxane. Alkyl linkers can include, but are not limited to, an n-propyl linker. Poly glycol chains include, but are not limited to, polyethylene glycol or polypropylene glycol. Poly glycol chains can comprise a mixture that provides an average chain length "n" of about "7.5". In certain embodiments, the average chain length "n" can vary from about 5 to about 14. Terminal groups can include, but are not limited to, alkyl groups such as a methyl group. Effective organosilicone compounds are believed to include, but are not limited to, trisiloxane ethoxylate surfactants or polyalkylene oxide modified heptamethyl trisiloxane. 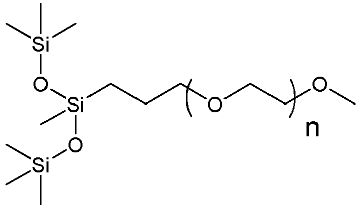 (Compound I: polyalkyleneoxide heptamethyltrisiloxane, average n=7.5).

In certain embodiments, an organosilicone preparation that comprises an organosilicone compound comprising a trisiloxane head group is used in the methods and compositions provided herein. In certain embodiments, an organosilicone preparation that comprises an organosilicone compound comprising a heptamethyltrisiloxane head group is used in the methods and compositions provided herein. In certain embodiments, an organosilicone composition that comprises Compound I is used in the methods and compositions provided herein. In certain embodiments, an organosilicone composition that comprises Compound I is used in the methods and compositions provided herein. In certain embodiments of the methods and compositions provided herein, a composition that comprises a polynucleotide molecule and one or more effective organosilicone compound in the range of about 0.015 to about 2 percent by weight (wt percent) (*e.g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) is used or provided.

Compositions include but are not limited components that are one or more polynucleotides essentially identical to, or essentially complementary to an EPSPS gene sequence (promoter, intron, exon, 5' untranslated region, 3' untranslated region), a transfer agent that provides for the polynucleotide to enter a plant cell, a herbicide that complements the action of the polynucleotide, one or more additional herbicides that further enhance the herbicide activity of the composition or provide an additional mode of action different from the complementing herbicide, various salts and stabilizing agents that enhance the utility of the composition as an admixture of the components of the composition.

In certain aspects, methods include one or more applications of a polynucleotide composition and one or more applications of a transfer agent for conditioning of a plant to permeation by polynucleotides. When the agent for conditioning to permeation is an organosilicone composition or compound contained therein, embodiments of the polynucleotide molecules are double-stranded RNA oligonucleotides, single-stranded RNA oligonucleotides, double-stranded RNA polynucleotides, single-stranded RNA polynucleotides, double-stranded DNA oligonucleotides, single-stranded DNA oligonucleotides, double-stranded DNA polynucleotides, single-stranded DNA polynucleotides, chemically modified RNA or DNA oligonucleotides or polynucleotides or mixtures thereof.

Compositions and methods are useful for modulating the expression of an endogenous EPSPS gene or transgenic EPSPS gene (for example, CP4 EPSPS, U.S. Pat. No. RE39,247 and 2mEPSPS, U.S. Pat. No. 6,040,497) gene in a plant cell. In various embodiments, an EPSPS gene includes coding (protein-coding or translatable) sequence, non-coding (non-translatable) sequence, or both coding and non-coding sequence. Compositions can include polynucleotides and oligonucleotides designed to target multiple genes, or multiple segments of one or more genes. The target gene can include multiple consecutive segments of a target gene, multiple non-consecutive segments of a target gene, multiple alleles of a target gene, or multiple target genes from one or more species.

Provided is a method for modulating expression of an EPSPS gene in a plant including (a) conditioning of a plant to permeation by polynucleotides and (b) treatment of the plant with the polynucleotide molecules, wherein the polynucleotide molecules include at least one segment of 18 or more contiguous nucleotides cloned from or otherwise identified from the target EPSPS gene in either anti-sense or sense orientation, whereby the polynucleotide molecules permeate the interior of the plant and induce modulation of the target gene. The conditioning and polynucleotide application can be performed separately or in a single step. When the conditioning and polynucleotide application are performed in separate steps, the conditioning can precede or can follow the polynucleotide application within minutes, hours, or days. In some embodiments more than one conditioning step or more than one polynucleotide molecule application can be performed on the same plant. In embodiments of the method, the segment can be cloned or identified from (a) coding (protein-encoding), (b) non-coding (promoter and other gene related molecules), or (c) both coding and non-coding parts of the target gene. Non-coding parts include DNA, such as promoter regions or the RNA transcribed by the DNA that provide RNA regulatory molecules, including but not limited to: introns, 5' or 3' untranslated regions, and microRNAs (miRNA), *trans*-acting siRNAs, natural anti-sense siRNAs, and other small RNAs with regulatory function or RNAs having structural or enzymatic function including but not limited to: ribozymes, ribosomal RNAs, t-RNAs, aptamers, and riboswitches.

The following examples are included to demonstrate examples of certain preferred embodiments.

### EXAMPLES

### Example 1. Polynucleotides related to the EPSPS gene sequences.

The target EPSPS gene polynucleotide molecules have been found that naturally occur in the genome of *Amaranthus palmeri, Amaranthus rudis, Amaranthus graecizans, Amaranthus hybridus, Amaranthus lividus, Amaranthus spinosus, Amaranthus thunbergii, Amaranthus viridis, Lolium multiflorum, Lolium rigidium, Ambrosia artemisiifolia, Ambrosia trifida, Euphorbia heterophylla, Kochia scoparia, Abutilon theophrasti, Sorghum halepense, Chenopodium album, Commelina diffusa, Conyza candensis, Digitaria sanguinalis,* and include molecules related to the expression of a polypeptide identified as an EPSPS, that include regulatory molecules, cDNAs comprising coding and noncoding regions of an EPSPS gene and fragments of the plant genes thereof as shown in Table 1. Additionally, the EPSPS gene coding sequence isolated from *Agrobacterium tumefaciens* that encodes for a glyphosate resistant EPSPS enzyme and that is commonly used to produce glyphosate resistant crop plants is shown in SEQ ID NO: 1 in Table 1.

Polynucleotide molecules were extracted from these plant species by methods standard in the field, for example, total RNA was extracted using Trizol Reagent (Invitrogen Corp, Carlsbad, CA Cat. No. 15596-018), following the manufacturer's protocol or modifications thereof by those skilled in the art of polynucleotide extraction that may enhance recover or purity of the extracted RNA. Briefly, start with 1 gram of ground plant tissue for extraction. Prealiquot 10 milliliters (mL) Trizol reagent to 15 mL conical tubes. Add ground powder to tubes and shake to homogenize. Incubate the homogenized samples for 5 minutes (min) at room temperature (RT) and then add 3 mL of chloroform. Shakes tubes vigorously by hand for 15-30 seconds(sec) and incubate at RT for 3 min. Centrifuge the tubes at 7,000 revolutions per minute (rpm) for 10 min at 4 degrees C. Transfer the aqueous phase to a new 1.5 mL tube and add 1 volume of cold isopropanol. Incubate the samples for 20-30 min at RT and centrifuge at 10,000 rpm for 10 min at 4 degrees C. Wash pellet with Sigma-grade 80 percent ethanol. Remove the supernatant and briefly air-dry the pellet. Dissolve the RNA pellet in approximately 200 microliters of DEPC treated water. Heat briefly at 65 degrees C to dissolve pellet and vortex or pipet to resuspend RNA pellet. Adjust RNA concentraiton to 1-2 microgram/microliter.

DNA was extracted using EZNA SP Plant DNA Mini kit (Omega Biotek, Norcross GA, Cat#D5511) and Lysing Matrix E tubes (Q-Biogen, Cat#6914), following the manufacturer's protocol or modifications thereof by those skilled in the art of polynucleotide extraction that may enhance recover or purity of the extracted DNA. Briefly, aliquot ground tissue to a Lysing Matrix E tube on dry ice, add 800µl Buffer SP1 to each sample, homogenize in a bead beater for 35-45sec, incubate on ice for 45-60 sec, centrifuge at ≥14000 rpm for 1min at RT, add 10 microliter RNase A to the lysate, incubate at 65°C for 10min, centrifuge for 1min at RT, add 280µl Buffer SP2 and vortex to mix, incubate the samples on ice for 5min, centrifuge at ≥10,000g for 10min at RT, transfer the supernatant to a homogenizer column in a 2ml collection tube, centrifuge at 10,000g for 2min at RT, transfer the cleared lysate into a 1.5ml microfuge tube, add 1.5 volumes Buffer SP3 to the cleared lysate, vortex immediately to obtain a homogeneous mixture, transfer up to 650µl supernatant to the Hi-Bind column, centrifuge at 10,000g for 1min, repeat, apply 100µl 65°C Elution Buffer to the column, centrifuge at 10,000g for 5min at RT.

Next-generation DNA sequencers, such as the 454-FLX (Roche, Branford, CT), the SOLiD (Applied Biosystems), and the Genome Analyzer (HiSeq2000, Illumina, San Diego, CA) were used to provide polynucleotide sequence from the DNA and RNA extracted from the plant tissues. Raw sequence data was assembled into contigs as illustrated in Table 1 and SEQ ID NO: 2-120. The contig sequence was used to identify trigger molecules that can be applied to the plant to enable regulation of the gene expression.

### Example 2. Polynucleotides related to the trigger molecules

The gene sequences and fragments of Table 1 were divided into 200 polynucleotide (200-mer) lengths with 25 polynucleotide overlapping regions (SEQ ID NO:121-3222). These polynucleotides are tested to select the most efficacious trigger regions across the length of any target sequence. The trigger polynucleotides are constructed as sense or anti-sense ssDNA or ssRNA, dsRNA, or dsDNA, or dsDNA/RNA hybrids and combined with an organosilicone based transfer agent to provide a polynucleotide preparation. The polynucleotides are combined into sets of two to three polynucleotides per set, using 4-8 nmol of each polynucleotide. Each polynucleotide set is prepared with the transfer agent and applied to a plant or a field of plants in combination with a glyphosate containing herbicide, or followed by a glyphosate treatment one to three days after the polynucleotide application, to determine the effect on the plant's susceptibility to glyphosate. The effect is measured as stunting the growth and/or killing of the plant and is measured 8-14 days after treatment with the polynucleotide set and glyphosate. The most efficacious sets are identified and the individual polynucleotides are tested in the same methods as the sets are and the most efficacious single 200-mer identified. The 200-mer sequence is divided into smaller sequences of 50-70-mer regions with 10-15 polynucleotide overlapping regions and the polynucleotides tested individually. The most efficacious 50-70-mer is further divided into smaller sequences of 25-mer regions with a 12 to 13 polynucleotide overlapping region and tested for efficacy in combination with glyphosate treatment. By this method it is possible to identify an oligonucleotide or several oligonucleotides that are the most efficacious trigger molecule to effect plant sensitivity to glyphosate or modulation of EPSPS gene expression. The modulation of EPSPS gene expression is determined by the detection of EPSPS siRNA moleclules specific to EPSPS gene or by an observation of a reduction in the amount of EPSPS RNA transcript produced relative to an untreated plant or by merely observing the anticipated phenotype of the application of the trigger with the glyphosate containing herbicide. Detection of siRNA can be accomplished, for example, using kits such as mirVana (Ambion, Austin TX) and mirPremier (Sigma-Aldrich, St Louis, MO).

The gene sequences and fragments of Table 1 were compared and 21-mers of contiguous polynucleotides were identified that have homology across the various EPSPS gene sequences (SEQ ID NO: 1-120). The purpose was to identify trigger molecules that are useful as herbicidal molecules or in combination with glyphosate herbicide enhance effective weed control across a broad range of weed species including glyphosate resistant weed biotypes. SEQ ID NO: 3223-3542 represent the 21-mers that are present in the EPSPS gene of at least eight of the weed species of Table 1. It is contemplated that additional 21-mers can be selected from the sequences of Table 1 that are specific for a single weed species or a few weeds species within a genus or trigger molecules that are at least 18 contiguous nucleotides, at least 19 contiguous nucleotides, at least 20 contiguous nucleotides or at least 21 contiguous nucleotides in length and at least 85 percent identical to an EPSPS gene sequence selected from the group consisting of SEQ ID NO:1-120 or fragments thereof. The 21-mer oligonucleotides are combined into a 6-12 oligonucleotide set and tested for efficacy against the broadest range of weed species in which the oligonucleotide is essentially identical or essentially complementary to the EPSPS gene sequence in the genome of the weed species. Efficacious sets are divided into smaller sets of 2-3 oligonucleotides and tested for efficacy. Each polynucleotide set is prepared with the transfer agent and applied to a plant or a field of plants in combination with a glyphosate containing herbicide, or followed by a glyphosate treatment one to three days after the oligonucleotide application, to determine the effect in the plant's susceptibility to glyphosate. The effect is measured as stunting the growth and/or killing of the plant and is measured 8-14 days after treatment with the polynucleotide set and glyphosate.

By this method it is possible to identify an oligonucleotide or several oligonucleotides that are the most efficacious trigger molecule to effect plant sensitivity to glyphosate or modulation of EPSPS gene expression. The modulation of EPSPS gene expression is determined by the detection of EPSPS siRNA moleclules specific to EPSPS gene or by an observation of a reduction in the amount of EPSPS RNA transcript produced relative to an untreated plant. Detection of siRNA can be accomplished, for example, using kits such as mirVana (Ambion, Austin TX) and mirPremier (Sigma-Aldrich, St Louis, MO).

### Example 3. Methods related to treating plants or plant parts with a topical mixture of the modified trigger oligonucleotide molecules.

Single stranded or double stranded DNA or RNA fragments in sense or antisense orientation or both were identified and mixed with a transfer agent and other components in the composition. This composition was topically applied to plants to effect expression of the target EPSPS genes in the specified plant to obtain the desired effect on growth or development.

In this example, growing *Amaranthus palmeri* plants were treated with a topically applied composition for inducing modulation of a target gene in a plant including (a) an agent for conditioning of a plant to permeation by polynucleotides and (b) polynucleotides including at least one polynucleotide strand including at least one segment of 17-25 contiguous nucleotides of the target gene in either anti-sense (AS) or sense (S) orientation. *Amaranthus palmeri* plants were treated with a topically applied adjuvant solution comprising dsRNA, ssDNA, and DNA/RNA hybrid polynucleotides shown in Table 2 (SEQ ID NO: 3544-3587, respectively) essentially homologous or essentially complementary to the *Amaranthus palmeri* EPSPS coding sequence. The polynucleotide sequences of the trigger molecules used in each treatment are shown in column 2. The trigger molecules, 5.2-RNA-M1 through M6 are modified for mismatch nucleotides (n) relative to 5.2.RNA-wt (wildtype). The type of polynucleotide for each trigger is shown in column 3, its' length in column 4 and the results observed in column 5. The results are expressed as a relative measure of activity, the tested oligonucleotide was either active, less active, or inactive in the bioassay.

A trigger sequence was identified to target the EPSPS promoter of glyphosate resistant *Amaranthus palmeri* and tests conducted to determine some activities of the trigger identified as AS83 (SEQ ID NO: 3670). The trigger sequence was made as ssDNA, dsDNA or dsRNA and various 3' and 5' deletions of AS83 were tested along with internal mismatch mutations. The following procedure was used for all assays described in this example. Approximately four-week old *Amaranthus palmeri* plants (glyphosate-resistant Palmer amaranth, "R-22") were used in this assay. Plants were treated with 0.1% Silwet L-77 solution freshly made with ddH2O. Two fully expanded leaves per plant (one cotyledon, one true leaf) awere treated with the polynucleotide/Silwet L-77 solution. Final concentration for each oligonucleotide or polynucleotide was 25 microM (in 0.01% Silwet L-77, 5 mM sodium phosphate buffer, pH 6.8) unless otherwise stated. Twenty microliters of the solution was applied to the top surface of each of the two pre-treated leaves to provide a total of 40 microliters (1 nmol oligonucleotide or polynucleotide) for each plant.

Spray solutions were prepared the same day as spraying. Single oligonucleotide molecules shown in Table 2 and Table 4 were applied at rates between 0.04 and 0.18 mg/ml in 20 mM potassium phosphate buffer (pH 6.8) are added to spray solutions 15 to 50 minutes before spraying. One-to-two-ml spray solutions were applied using a custom low-dead-volume sprayer ("milli applicator") at 8-30 gpa (gallons per acre) to one-to-four inch tall plants. Treated plants were place in a greenhouse set for either a 26.7/21.1 °C or 29.4/21.1°C 14/10 hour temperature and supplemental light schedule. The amount of response relative to unsprayed treatments was collected at various time intervals up to 21 days after treatment.

The current default spray nozzle used for all applications made with the track sprayer is the Turbo Teejet air induction nozzle (015) nozzle with air pressure set at a minimum of 20 psi (160kpa). The height of the spray nozzle was 16-18 inches above top of plant material. Treatments were made when plants reach the desired size, height or leaf stage.

Application rates are chosen so as to achieve percent control ratings in the range of 50% at the lowest rate to 90% control at the highest rate. The rates in this control range provide the best possible efficacy comparisons among formulations, allowing separation of relative performance of test samples. The rate of glyphosate used in these studies is typically held constant at 1680g ae/ha (grams acid equivalent/hectare). On occasion lower or higher rates may be necessary depending on test objectives. The rate structure used for a given test will be dependent on the environmental conditions at the time of spray application (time of year), the plant species being treated (highly susceptible or tough to kill) and age (or size) of plants to be treated.

These results illustrated in Table 2 shows that dsRNA, dsDNA and ssDNA were effective oligonucleotides trigger molecules for activity against EPSPS gene exon coding sequence and noncoding (promoter) sequence. Generally, 3 mismatches in a 21-mer or about 85 percent sequence homology can be tolerated, oligonucleotides shorter than 21 appear to have less activity in this assay Other modifications, such as the addition of some 3' synthetic nucleotides (ddC and IdT) did not seem to be tolerated in this bioassay.

**Table 2. Various polynucleotide types and modified sequence homologies**

| **1. Oligo name** | **2. Sequence (AS strand, unless otherwise indicated)** | **3. Type** | **4.length** | **5.Activity** |
|---|---|---|---|---|
| 5.2-RNA-wt | GTC ATA GCA ACA TCT GGC ATT | dsRNA | 21 | active |
| 5.2-DNA-wt | GTC ATA GCA ACA TCT GGC ATT | dsDNA | 21 | active |
| 5.2-ssDNA-S | AAT GCC AGA TGT TGC TAT GAC | ssDNA | 21 | less active |
| 5.2-ssDNA-AS | GTC ATA GCA ACA TCT GGC ATT | ssDNA | 21 | less active |
| 5.2-sDNA/asRNA | GTC ATA GCA ACA TCT GGC ATT | DNA/RNA hybrid | 21 | inactive |
| 5.2-asDNA/sRNA | GTC ATA GCA ACA TCT GGC ATT | DNA/RNA hybrid | 21 | inactive |
| 5.2-RNA-5'-20 | TC ATA GCA ACA TCT GGC ATT | dsRNA | 20 | active |
| 5.2-RNA-5'-19 | C ATA GCA ACA TCT GGC ATT | dsRNA | 19 | less active |
| 5.2-RNA-5'-18 | ATA GCA ACA TCT GGC ATT | dsRNA | 18 | inactive |
| 5.2-RNA-M1 | GTC ATA GCA ACA TCT GGC ATa | dsRNA | 21 | active |
| 5.2-RNA-M2 | GTC ATA GCA ACA TCT GGC Aaa | dsRNA | 21 | active |
| 5.2-RNA-M3 | GTC ATA GCA AgA TCT GGC ATT | dsRNA | 21 | inactive |
| 5.2-RNA-M4 | GTC ATA GCA tgt TCT GGC ATT | dsRNA | 21 | less active |
| 5.2-RNA-M5 | GTC Aat GCA ACA TCT GGC ATT | dsRNA | 21 | active |
| 5.2-RNA-M6 | GTC ATA GCA ACA TCT ccC ATT | dsRNA | 21 | active |
| 5.2-RNA-3'ddC | GTC ATA GCA ACA TCT GGC ATT-3'ddC | dsRNA | 22 | inactive |
| 5.2-RNA-3'IdT | GTC ATA GCA ACA TCT GGC ATT-3'IdT | dsRNA | 22 | inactive |

| **3**' **Deletion Analysis** | | | | |
|---|---|---|---|---|
| AS83-DNA-25-wt | CTC TTT GTT TTT CTT CTG CCA ATT T | ssDNA | 25 | active |
| AS83-DNA-24-3'D | CTC TTT GTT TTT CTT CTG CCA ATT | ssDNA | 24 | active |
| AS83-DNA-23-3'D | CTC TTT GTT TTT CTT CTG | ssDNA | 23 | active |
| | CCA AT | | | |
| AS83-DNA-22-3'D | CTC TTT GTT TTT CTT CTG CCA A | ssDNA | 22 | active |
| AS83-DNA-21-3'D | CTC TTT GTT TTT CTT CTG CCA | ssDNA | 21 | active |
| AS83-DNA-20-3'D | CTC TTT GTT TTT CTT CTG CC | ssDNA | 20 | active |
| AS83-DNA-19-3'D | CTC TTT GTT TTT CTT CTG C | ssDNA | 19 | inactive |
| AS83-DNA-18-3'D | CTC TTT GTT TTT CTT CTG | ssDNA | 18 | inactive |
| AS83-DNA-17-3'D | CTC TTT GTT TTT CTT CT | ssDNA | 17 | inactive |

| **5**' **Deletion Analysis** | | | | |
|---|---|---|---|---|
| AS83-DNA-24-5'D | TCT TTG TTT TTC TTC TGC CAA TTT | ssDNA | 24 | active |
| AS83-DNA-23-5'D | CTT TGT TTT TCT TCT GCC AAT TT | ssDNA | 23 | active |
| AS83-DNA-22-5'D | TTT GTT TTT CTT CTG CCA ATT T | ssDNA | 22 | active |
| AS83-DNA-21-5'D | TTG TTT TTC TTC TGC CAA TTT | ssDNA | 21 | active |
| AS83-DNA-20-5'D | TTG TTT TTC TTC TGC CAA TT | ssDNA | 20 | active |
| AS83-DNA-19-5'D | TTG TTT TTC TTC TGC CAA T | ssDNA | 19 | less active |
| AS83-DNA-18-5'D | TTG TTT TTC TTC TGC CAA | ssDNA | 18 | inactive |
| AS83-DNA-17-5'D | TTG TTT TTC TTC TGC CA | ssDNA | 17 | inactive |

| **Mutational Analysis** | | | | |
|---|---|---|---|---|
| AS83-DNA-5'M1 | gTC TTT GTT TTT CTT CTG CCA ATT T | ssDNA | 25 | less active |
| AS83-DNA-5'M2 | gaC TTT GTT TTT CTT CTG CCA ATT T | ssDNA | 25 | less active |
| AS83-DNA-M3 | CTC TTT GTT TTT gTT CTG CCA ATT T | ssDNA | 25 | less active |
| AS83-DNA-M4 | CTC TTT GTT TT*a ga*T CTG CCA ATT T | ssDNA | 25 | less active |
| AS83-DNA-M5 | CTC TTT caa TTT CTT CTG CCA ATT T | ssDNA | 25 | less active |
| AS83-DNA-M6 | CTC TTT GTT TTT CTT Cac gCA ATT T | ssDNA | 25 | less active |
| AS83-DNA-3'M1 | CTC TTT GTT TTT CTT CTG CCA ATT *a* | ssDNA | 25 | less active |
| AS83-DNA-3'M2 | CTC TTT GTT TTT CTT CTG CCA AT*a a* | ssDNA | 25 | inactive |
| AS83-DNA-3'ddC | CTC TTT GTT TTT CTT CTG CCA ATT T-3'ddC | ssDNA | 26 | inactive |
| AS83-DNA-3'InvdT | CTC TTT GTT TTT CTT CTG CCA ATT T-3'InvdT | ssDNA | 26 | inactive |

### Example 4. Identification of effective trigger polynucleotides

One non-limiting example of a method for selecting a polynucleotide for use in a composition for topical application to the surface of a parent plant involves the mapping of efficacious oligonucleotide or polynucleotide sequences (or segments of sequences) using a whole-gene (or full-length reference sequence) tiling array approach. The available full-length reference sequence is divided into "tiling sequences" or segments of 25 contiguous nucleotides along the entire length of the available sequence. For convenience, an Excel template was developed to allow convenient generation of sense and anti-sense tiling sequences for any given full-length reference sequence, providing as output a list of sense and anti-sense sequences for submission to oligonucleotide synthesis providers such as IDT (Integrated DNA Technologies, Coralville, IA). Oligonucleotides corresponding to each 25-mer tiling sequence (in sense, anti-sense, or both sense and anti-sense orientation) are synthesized for efficacy screening. Oligonucleotides are screened in sets. It is clear to one skilled in the art that the tiling sequences can be of sizes other than 25 nucleotides (such as about 18, 19, 20, 21, 22, 23, or 24 nucleotides, or larger than 25 nucleotides), that these tiling sequences can be designed to be contiguous segments with no overlap or to overlap adjacent segments, and that such tiling sequences can be grouped into sets of any size. For example, sets of five individual oligonucleotides are pooled into a single polynucleotide composition using 20 mM phosphate buffer and 2 percent w/v ammonium sulfate and 1 percent Silwet L-77, and topically applied to plants at a rate known to be efficacious for the plant species of interest (e.g., 4 nanomoles per plant). Those oligonucleotide sets showing better efficacy are then re-screened by testing the individual component oligonucleotides for efficacy.

A specific example of selecting a polynucleotide for use in a composition for topical application to the surface of a parent plant follows. An EPSPS promoter 1302 nucleotide sequence was identified from genomic sequence of Palmer amaranth (*Amaranthus palmeri*) as having the sequence SEQ ID NO: 3543. A 1152 nucleotide segment of the 1302 nucleotides EPSPS promoter sequence was used in this example.

The 1152-nt EPSPS promoter sequence was "tiled" (*i*.*e.,* the full-length sequence covered by overlapping shorter sequences) by 25-mer anti-sense (AS) and sense (S) ssDNAs. A total of 96 25-mer ssDNA oligonucleotides were designed and grouped into 16 sets of 6 ssDNA oligonucleotides each (each set covering 150 contiguous nucleotides of the promoter sequence). The oligonucleotides were synthesized by IDT in 96-well plate format. Oligonucleotide sequences are provided in Table 3 (SEQ ID NO: 3588-3779). The oligonucleotides in a given set consisted of six contiguous sequences (in terms of their position within the 1152-nt full-length sequence) where each oligonucleotide did not overlap the adjacent oligonucleotide(s).

**Table 3. Polynucleotides for targeting Amaranthus palmeri EPSPS promoter**

| Name | Antisense Sequence | SEQ ID NO: | Name | Sense Sequence | SEQ ID NO: |
|---|---|---|---|---|---|
| AS1 | cgaatcaaaggaaaaagttatccaa | 3588 | S_1 | ttggataactttttcctttgattcg | 3684 |
| AS2 | aataatccgattcgaatcaaaggaa | 3589 | S_3 | gaatcggattatttttaatacagta | 3686 |
| AS3 | tactgtattaaaaataatccgattc | 3590 | S_5 | attatgaactgatttaatgaaagtg | 3688 |
| AS4 | atcagttcataatactgtattaaaa | 3591 | S_7 | ggaggaagtttcaatttttaaagtt | 3690 |
| AS5 | cactttcattaaatcagttcataat | 3592 | S_9 | tgtaggtgtaatgttttctcatttt | 3692 |
| AS6 | tgaaacttcctccactttcattaaa | 3593 | S_11 | tggatatgaaagtggaggaagtttc | 3694 |
| AS7 | aactttaaaaattgaaacttcctcc | 3594 | S_2 | ttcctttgattcgaatcggattatt | 3685 |
| AS8 | cattacacctacaactttaaaaatt | 3595 | S_4 | ttttaatacagtattatgaactgat | 3687 |
| AS9 | aaaatgagaaaacattacacctaca | 3596 | S_6 | tttaatgaaagtggaggaagtttca | 3689 |
| AS10 | actttcatatccaaaatgagaaaac | 3597 | S_8 | aatttttaaagttgtaggtgtaatg | 3691 |
| AS11 | gaaacttcctccactttcatatcca | 3598 | S_10 | gttttctcattttggatatgaaagt | 3693 |
| AS12 | tgattcgaaattgaaacttcctcca | 3599 | S_12 | tggaggaagtttcaatttcgaatca | 3695 |
| AS13 | aactggcaaacatgattcgaaattg | 3600 | S_13 | caatttcgaatcatgtttgccagtt | 3696 |
| AS14 | attcattgaatcaactggcaaacat | 3601 | S_15 | tgattcaatgaatgctcttggaaat | 3698 |
| AS15 | atttccaagagcattcattgaatca | 3602 | S_17 | tgaccaagagttcaaggcttcttgt | 3700 |
| AS16 | gaactcttggtcatttccaagagca | 3603 | S_19 | ttataaaacatttcaattttgatct | 3702 |
| AS17 | acaagaagccttgaactcttggtca | 3604 | S_21 | taagaatgaactatttagaacttaa | 3704 |
| AS18 | aaatgttttataacaagaagccttg | 3605 | S_23 | aagtaattaaattattagttataac | 3706 |
| AS19 | agatcaaaattgaaatgttttataa | 3606 | S_14 | atgtttgccagttgattcaatgaat | 3697 |
| AS20 | tagttcattcttagatcaaaattga | 3607 | S_16 | tgctcttggaaatgaccaagagttc | 3699 |
| AS21 | ttaagttctaaatagttcattctta | 3608 | S_18 | caaggcttcttgttataaaacattt | 3701 |
| AS22 | aatttaattactttaagttctaaat | 3609 | S_20 | tcaattttgatctaagaatgaacta | 3703 |
| AS23 | gttataactaataatttaattactt | 3610 | S_22 | atttagaacttaaagtaattaaatt | 3705 |
| AS24 | atttttttataagttataactaata | 3611 | S_24 | tattagttataacttataaaaaaat | 3707 |
| AS25 | ggttaaaattgaatttttttataag | 3612 | S_25 | cttataaaaaaattcaattttaacc | 3708 |
| AS26 | ttataaatttaaggttaaaattgaa | 3613 | S_27 | cttaaatttataaattatgacctta | 3710 |
| AS27 | taaggtcataatttataaatttaag | 3614 | S_29 | aaaaagatcaagtattgaacgcata | 3712 |
| AS28 | acttgatctttttaaggtcataatt | 3615 | S_31 | atttagaaaaattataattcggctt | 3714 |
| AS29 | tatgcgttcaatacttgatcttttt | 3616 | S_33 | tatcagtctcatattgagacggtct | 3716 |
| AS30 | aatttttctaaatatgcgttcaata | 3617 | S_35 | Tcgtccaagacaagttgtatcattt | 3718 |
| AS31 | aagccgaattataatttttctaaat | 3618 | S_26 | ttcaattttaaccttaaatttataa | 3709 |
| AS32 | tatgagactgataagccgaattata | 3619 | S_28 | aattatgaccttaaaaagatcaagt | 3711 |
| AS33 | agaccgtctcaatatgagactgata | 3620 | S_30 | tattgaacgcatatttagaaaaatt | 3713 |
| AS34 | Ttgtcttggacgagaccgtctcaat | 3621 | S_32 | tataattcggcttatcagtctcata | 3715 |
| AS35 | aaatgatacaacttgtcttggacga | 3622 | S_34 | attgagacggtctcgtccaagacaA | 3717 |
| AS36 | atttgattatataaatgatacaact | 3623 | S_36 | Agttgtatcatttatataatcaaat | 3719 |
| AS37 | actcataattatatttgattatata | 3624 | S_37 | Tatataatcaaatataattatgagt | 3720 |
| AS38 | ctacatgaatacactcataattata | 3625 | S_39 | Tgtattcatgtaggtttcaacttta | 3722 |
| AS39 | taaagttgaaacctacatgaataca | 3626 | S_41 | Aaagcctaggtgaaagatatgttgt | 3724 |
| AS40 | tcacctaggctttaaagttgaaacc | 3627 | S_43 | Tagcatctttgtgaaagtcagccta | 3726 |
| AS41 | acaacatatctttcacctaggcttt | 3628 | S_45 | Ataacttggttctaaaattttgaag | 3728 |
| AS42 | cacaaagatgctacaacatatcttt | 3629 | S_47 | Gcataaccatatagtccctcgaatt | 3730 |
| AS43 | taggctgactttcacaaagatgcta | 3630 | S_38 | Tataattatgagtgtattcatgtag | 3721 |
| AS44 | agaaccaagttataggctgactttc | 3631 | S_40 | Ggtttcaactttaaagcctaggtga | 3723 |
| AS45 | cttcaaaattttagaaccaagttat | 3632 | S_42 | Aaagatatgttgtagcatctttgtg | 3725 |
| AS46 | tatatggttatgcttcaaaatttta | 3633 | S_44 | Gaaagtcagcctataacttggttct | 3727 |
| AS47 | aattcgagggactatatggttatgc | 3634 | S_46 | Taaaattttgaagcataaccatata | 3729 |
| AS48 | acaacttgaatgaattcgagggact | 3635 | S_48 | Agtccctcgaattcattcaagttgt | 3731 |
| AS49 | aaagtaaattggacaacttgaatga | 3636 | S_49 | Tcattcaagttgtccaatttacttt | 3732 |
| AS50 | ggcaagtataaaaaagtaaattgga | 3637 | S_51 | Ttttatacttgccgagacaacattt | 3734 |
| AS51 | aaatgttgtctcggcaagtataaaa | 3638 | S_53 | Ttaaacccttaatatttctaattaa | 3736 |
| AS52 | attaagggtttaaaatgttgtctcg | 3639 | S_55 | Atcttaattaaaaattatgaaaatt | 3738 |
| AS53 | ttaattagaaatattaagggtttaa | 3640 | S_57 | Ttgatattaataatctttgtattga | 3740 |
| AS54 | ttttaattaagattaattagaaata | 3641 | S_59 | Aaacgaatttaacaagatctcacat | 3742 |
| AS55 | aattttcataatttttaattaagat | 3642 | S_50 | Tccaatttacttttttatacttgcc | 3733 |
| AS56 | ttattaatatcaaattttcataatt | 3643 | S_52 | Cgagacaacattttaaacccttaat | 3735 |
| AS57 | tcaatacaaagattattaatatcaa | 3644 | S_54 | Tatttctaattaatcttaattaaaa | 3737 |
| AS58 | gttaaattcgtttcaatacaaagat | 3645 | S_56 | Aattatgaaaatttgatattaataa | 3739 |
| AS59 | atgtgagatcttgttaaattcgttt | 3646 | S_58 | Atctttgtattgaaacgaatttaac | 3741 |
| AS60 | taaaacatagtcatgtgagatcttg | 3647 | S_60 | Caagatctcacatgactatgtttta | 3743 |
| AS61 | taatctataagttaaaacatagtca | 3648 | S_61 | Tgactatgttttaacttatagatta | 3744 |
| AS62 | ttgtattttttttaatctataagtt | 3649 | S_63 | Aaaaaaaatacaaattaagagtgat | 3746 |
| AS63 | atcactcttaatttgtatttttttt | 3650 | S_65 | Taagtgaatagtgccccaaaacaaa | 3748 |
| AS64 | cactattcacttatcactcttaatt | 3651 | S_67 | Atgggacaacttagatgaattggag | 3750 |
| AS65 | tttgttttggggcactattcactta | 3652 | S_69 | Ggtaatattaggtagcaagtgatct | 3752 |
| AS66 | taagttgtcccatttgttttggggc | 3653 | S_71 | Tagcaagtgatcactttaacatcaa | 3754 |
| AS67 | ctccaattcatctaagttgtcccat | 3654 | S_62 | Aacttatagattaaaaaaaatacaa | 3745 |
| AS68 | acctaatattacctccaattcatct | 3655 | S_64 | Aattaagagtgataagtgaatagtg | 3747 |
| AS69 | agatcacttgctacctaatattacc | 3656 | S_66 | Gccccaaaacaaatgggacaactta | 3749 |
| AS70 | tgatcacttgctagatcacttgcta | 3657 | S_68 | Agatgaattggaggtaatattaggt | 3751 |
| AS71 | ttgatgttaaagtgatcacttgcta | 3658 | S_70 | Tagcaagtgatctagcaagtgatca | 3753 |
| AS72 | aagtgatcaattttgatgttaaagt | 3659 | S_72 | Actttaacatcaaaattgatcactt | 3755 |
| AS73 | atttgaacctataagtgatcaattt | 3660 | S_73 | Aaattgatcacttataggttcaaat | 3756 |
| AS74 | gtaaaagtttcaatttgaacctata | 3661 | S_75 | Ttgaaacttttactttaattgatat | 3758 |
| AS75 | atatcaattaaagtaaaagtttcaa | 3662 | S_77 | Tgtttaaatactactttaaattgaa | 3760 |
| AS76 | tagtatttaaacatatcaattaaag | 3663 | S_79 | Aattgatatttttaaggtcaaaatt | 3762 |
| AS77 | ttcaatttaaagtagtatttaaaca | 3664 | S_81 | Tgaaacctttaagattataattgaa | 3764 |
| AS78 | aaaaatatcaatttcaatttaaagt | 3665 | S_83 | Aaattggcagaagaaaaacaaagag | 3766 |
| AS79 | aattttgaccttaaaaatatcaatt | 3666 | S_74 | Tataggttcaaattgaaacttttac | 3757 |
| AS80 | cttaaaggtttcaattttgacctta | 3667 | S_76 | Ctttaattgatatgtttaaatacta | 3759 |
| AS81 | ttcaattataatcttaaaggtttca | 3668 | S_78 | Actttaaattgaaattgatattttt | 3761 |
| AS82 | cttctgccaattttcaattataatc | 3669 | S_80 | Taaggtcaaaattgaaacctttaag | 3763 |
| AS83 | ctctttgtttttcttctgccaattt | 3670 | S_82 | Gattataattgaaaattggcagaag | 3765 |
| AS84 | cttatattctttctctttgtttttc | 3671 | S_84 | Gaaaaacaaagagaaagaatataag | 3767 |
| AS85 | caatttgcgtgtcttatattctttc | 3672 | S_85 | Gaaagaatataagacacgcaaattg | 3768 |
| AS86 | agtagatcggtacaatttgcgtgtc | 3673 | S_87 | Gtaccgatctactcttatttcaatt | 3770 |
| AS87 | aattgaaataagagtagatcggtac | 3674 | S_89 | Tttgagacggtctcgcccaagacta | 3772 |
| AS88 | agaccgtctcaaaattgaaataaga | 3675 | S_91 | Agatgttcggtcatcctacaccaac | 3774 |
| AS89 | tagtcttgggcgagaccgtctcaaa | 3676 | S_93 | Ccccaaaaaattcaacaacaaagtc | 3776 |
| AS90 | tgaccgaacatctagtcttgggcga | 3677 | S_95 | Cttataatgattccctctaatctac | 3778 |
| AS91 | gttggtgtaggatgaccgaacatct | 3678 | S_86 | Gacacgcaaattgtaccgatctact | 3769 |
| AS92 | gaattttttggggttggtgtaggat | 3679 | S_88 | Tcttatttcaattttgagacggtct | 3771 |
| AS93 | gactttgttgttgaattttttgggg | 3680 | S_90 | Tcgcccaagactagatgttcggtca | 3773 |
| AS94 | gaatcattataagactttgttgttg | 3681 | S_92 | Atcctacaccaaccccaaaaaattc | 3775 |
| AS95 | gtagattagagggaatcattataag | 3682 | S_94 | Caacaacaaagtcttataatgattc | 3777 |
| AS96 | gtgtagactgtagtagattagaggg | 3683 | S_96 | Ccctctaatctactacagtctacac | 3779 |

Oligonucleotide sets assigned an even number n contain oligonucleotides with a sequence shifted by 12 or 13 nucleotides (nt) relative to the 3'end of the oligonucleotides in sets assigned a number equal to (*n* - 1). For example, the oligonucleotide sequences in set number 2 have a sequence shifted by 12 or 13 nt relative to the 3' end of the oligonucleotides in set number 1.

The ssDNA oligonucleotides were formulated as 100 micromolar (per oligonucleotide) mixtures (each consisting of a set of 6 oligonucleotides) in 20 millimolar phosphate buffer (pH 7.0), 2% ammonium sulfate, 1% Silwet® L-77, and were hand applied by pipetting to the surface of four fully expanded source leaves of glyphosate-resistant Palmer amaranth (*Amaranthus palmeri* R-22) plants. Each leaf received 10 microliters of 100 micromolar ssDNA solution (a total of 1 nanomole per oligonucleotide per leaf for a total 4 nanomole per oligonucleotide per plant). Silwet-containing buffer without oligonucleotides was applied as a negative control. A composition of four EPSPS short dsRNA1, 3, 4 and 5 (see Example 6) were applied at 4 nm each oligonucleotide per plant as positive control. The Palmer plants were then sprayed with 2X WeatherMax (1.5 lb/ac) at either 2 or 3-day after oligos treatment.

The first round of efficacy testing showed that sets 8 and 13 gave better herbicidal control of Palmer amaranth (for both sense and anti-sense strands). A second round of efficacy testing used the 12 individual oligonucleotides in sets 8 and 13 and showed that five individual ssDNA oligonucleotides numbered 44, 48, 79, 81, and 83 gave better herbicidal control of Palmer amaranth than the other seven ssDNA oligonucleotides. These five ssDNA oligonucleotides were individually tested at 16 nmol/plant followed by 2X WMax on Palmer R-22 plants. The treated Palmer amaranth plants were observed ten days after treatment and showed that ssDNA oligonucleotides numbers 79 (SEQ ID NO: 3666), 81(SEQ ID NO: 3668), and 83(SEQ ID NO: 3670) gave 95, 98 and 99 percent control respectively when applied in combination with dsRNA5 (EPSPS) and dsRNATIF1 (SEQ ID NO: 3780).

Further experimental testing of the AS83 trigger that targets the EPSPS promoter in Palmer R-22 was conducted in which the AS83 trigger was used to make ssDNA, dsRNA (SEQ ID NO:3789) and dsDNA. These AS83 molecules were tested as described in Exampe 3 and the results shown in Table 4 determined that all of the molecular forms of AS83 were active in making the Palmer R-22 plant sensitive to glyphosate.

**Table 4. EPSPS promoter AS83 trigger molecules**

| **Oligo Name** | **Sequence (AS/bottom strand)** | **Type** | **Size** | **Activity** |
|---|---|---|---|---|
| AS83-DNA-wt- | CTC TTT GTT TTT CTT CTG CCA ATT T | ssDNA | 25 | active |
| AS83-RNA-wt- | CUC UUU GUU UUU CUU CUG CCA AUU U | dsRNA | 25 | active |
| AS83-DNA-blunt | CTC TTT GTT TTT CTT CTG CCA ATT T | dsDNA | 25 | active |

### Example 5

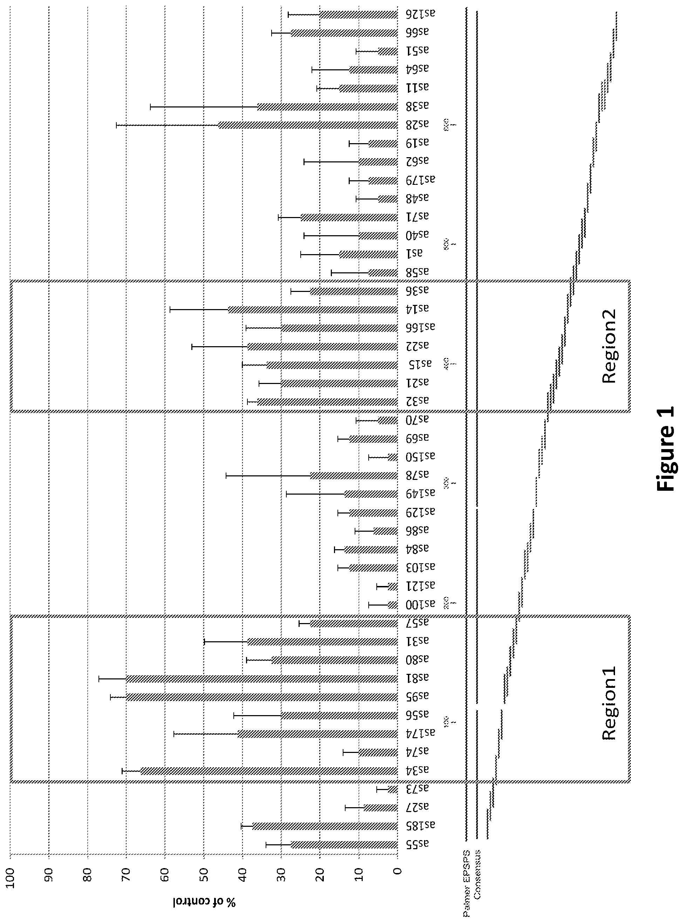

Tiling of tigger oligonucleotides was conducted on a Palmer amaranth EPSPS coding region using a similar testing protocol as described in Example 3. In this test, approximately 700 base pairs of coding region were used to select 46 individual antisense ssDNA oligonucleotides each 25 nucleotides long. These were applied to Palmer amaranth plants (R-22) at 12nmole per oligonucleotide per plant, followed by 2X WeatherMax 2 days after oligonucleotide treatment later.. The plants were scored for glyphosate effect on growth. As shown in Figure 1, there were two regions identified in the coding sequence where many of the trigger molecules were able to provide a glyphosate sensitive phenotype to the treated plants, these are identified by the boxes in Figure 1. The 5' region (Region 1, SEQ ID NO:3787) is approximately 150 nucleotides including and between antisense oligo 34 (SEQ ID NO: 3781) and 57 (SEQ ID NO: 3782) and the 3' region (Region 2, SEQ ID NO:3788) is approximately 100 nucleotides including and between antisense oligo 32 (SEQ ID NO:3783) and oligo 36 (SEQ ID NO:3784). The triggers plus glyphosate provided 30-70 percent and 25-45 percent control in the 5' region and in the 3' region, respectively. Additional trigger polynucleotides in these regions include oligo 81 (SEQ ID NO: 3785) and oligo 95 (SEQ ID NO: 3786). It is contemplated that additional trigger molecules can be identified in these regions and combinations of triggers would be useful to provide a high level of glyphosate sensitivity.

### Example 6. Effects on transgenic herbicide tolerant plants

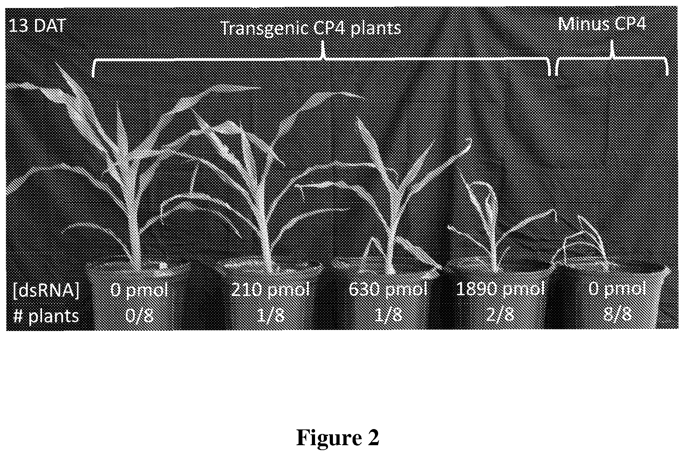

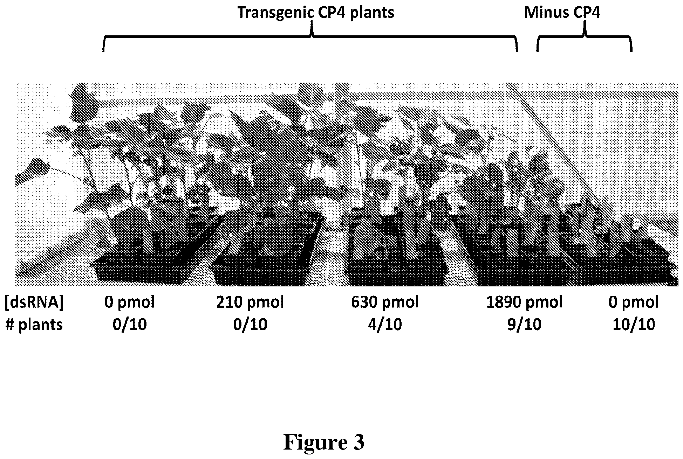

This example demonstrates that the topical application of a polynucleotide trigger molecule can be used to make transgenic herbicide tolerant crops sensitive to the herbicide for which they were engineered to be tolerant. In this example, a gene coding sequence for *Agrobacterium tumefaciens* CP4 EPSPS (SEQ ID NO: 1) was targeted with two dsRNA trigger molecules referred to as CP4-12 (82-462 of SEQ ID NO:1) a 381 polynucleotide, and CP4-34 (594-1043 of SEQ ID NO:1) a 450 polynucleotide. Corn and cotton plants that were transformed with the CP4 EPSPS gene and are resistant to glyphosate were planted in pots in a greenhouse along with negative isolines for each and grown to the first true leaf emergence stage then treated with a trigger solution containing 0.5% Silwet L77, 2% ammonium sulfate, 20 mM Na Phoshpate (pH 6.8) at different rates of trigger amount, 0 picomoles (pmol), 210 pmol, 630 pmol and 1890 pmol. For each replication (8-10 plants), two fully expanded cotyledons were treated by pipette with 50 microliters of trigger solution each, then sprayed two-three days later with 1.5 a.e.lb/acre) of RoundUp™ Ultra (glyphosate, Monsanto, St Louis, MO). The cotton and corn plants were scored for stunting and injury 7-16 days after spray treatment. Figures 2 and 3 show the corn and cotton plant results, respectively. The corn plants in Figure 2 shows the number of treated plants that showed glyphosate injury after treatment with the trigger polynucleotides and glyphosate, injury was observed as dead or damaged terminal leaves 2-4 days after RoundUp treatment, and stunting was evident 7-14 days after RoundUp treatment. The nontransgenic control is labeled "Minus CP4", these plant were killed by the RoundUp treatment. Figure 3 shows the results of glyphosate tolerant cotton plants treated with the trigger polynucleotides and glyphosate, symptoms observed on the cotton plants were severe stunting and death of the apical meristem. These results demonstrate that topical treatment with a trigger polynucleotide can be used to effect a herbicide tolerant trait in a transgenic herbicide tolerant crop plant.

### Example 7. Enhancement with the addition of non-EPSPS herbicides

This example demonstrates that the addition of herbicides with a mode of action different than glyphosate that enhance the effect of the treatment comprising glyphosate , an EPSPS trigger polynucleotides, and an essential gene (transcription initiation factor, TIF) trigger polynucleotide. Glyphosate is applied as a Roundup WeatherMAX® formulation (RU Wmax, Monsanto, St Louis, MO) at 2X (1.5 pounds acid equivalent/acre), 4X and 8X in a field test plot infested with glyphosate resistant *A. palmeri.* Clarity® (Diglycolamine salt, BASF) is a dicamba formulation applied at 0.25 pounds/acre (lb/ac) is equal to half of the recommended use rate for broadleaf weed control. The polynucleotides are all dsRNA, 4001 is mixture of the following four *A. palmeri* EPSPS dsRNA trigger polynucleotides: dsRNA1: sense strand sequence CUACCAUCAACAAUGGUGUCC (1479-1499 of SEQ ID NO: 10) and complementary anti-sense strand, and dsRNA3: sense strand GUCGACAACUUGCUGUAUAGU (4241-4261 of SEQ ID NO: 10) and complementary anti-sense strand, and dsRNA4: sense strand GGUCACCUGGACAGAGAAUAG(9919-9939 of SEQ ID NO: 10) and complementary anti-sense strand, and dsRNA5: sense strand AAUGCCAGAUGUUGCUAUGAC (10015-10035 of SEQ ID NO: 10) and complementary anti-sense strand and one *A. palmeri* TIF dsRNA trigger polynucleotide (dsRNATIF1: sense strand GCACAAAUGUAAAUAAACCGUCUCC (SEQ ID NO: 3780) and complementary anti-sense strand), 4002 is mixture of one EPSPS dsRNA trigger polynucleotide (dsRNA5) and one *A. palmeri* TIF dsRNA trigger polynucleotide (dsRNATIF1). The composition of the herbicides and polynucleotides also contain one percent Silwet L77.

The treatments of the field plots containing glyphosate resistant *A. palmeri* plants (mostly 4-6 inches tall) with compositions shown in Table 6 with 4 replications per treatment at a spray volume of 10 gallons per acre, the total polynucleotide concentration was in the composition was approximately 160nmol. The treated glyphosate resistant *A. palmeri* were scored for percent injury between 10-14 days post treatment. The results show that glyphosate (RU Wmax) was not effective in controlling this population of resistant *A. palmeri* even at 8X the recommended field rates, 52.5 percent. The addition of the 4001 and 4002 polynucleotides substantially increased the observed glyphosate percent injury, 83.75 and 72.5 percent respectively. The treatments that also included 0.25 lb/ac Clarity (dicamba) increased the injury rate to 95.75 percent when included in the composition with the 4001 trigger polynucleotides and to 93.25 percent when included in the composition with the 4002 trigger polynucleotides. The RU Wmax and Clarity alone showed a 83.75 percent injury rate on the glyphosate resistant *A. palmeri.*

**Table 5. Addition of Dicamba to glyphosate and EPSPS and essential gene trigger polynucleotides enhances injury rates to glyphosate resistant A. palmeri.**

| Treatment | Percent injury Mean | std err |
|---|---|---|
| RU Wmax 2X | 28.75 | 12.045 |
| RU Wmax 4X | 30 | 4.291 |
| RU Wmax 8X | 52.5 | 11.219 |
| 2X RU Wmax + 4001 | 83.75 | 3.1 |
| 2X RU Wmax + 4001 + 0.25 lb Clarity | 95.75 | 4.095 |
| 2X RU Wmax + 4002 | 72.5 | 3.067 |
| 2X RU Wmax + 4002 + 0.25 lb Clarity | 93.25 | 3.513 |
| 2X RU Wmax + 0.25 lb Clarity | 83.75 | 6.221 |

A greenhouse test was conducted to determine the effect of a composition containing 2,4-D herbicide, an EPSPS dsRNA, an essential gene dsRNA and a glyphosate herbicide. The polynucleotides used in the test was 4002 which is a mixture of 1 EPSPS dsRNA trigger polynucleotide (dsRNA5) and 1 *A. palmeri* TIF dsRNA trigger polynucleotide (dsRNATIF1), at a concentration of 80nm applied with a 9501E nozzle at 93 L/ha (liters/hectare). Roundup WeatherMax® was the glyphosate herbicide and applied at the 2X rate. The 2,4-D herbicide is 2,4D amine (dimethylamine salt) at a concentration of 3.8 lb/gal and tested at 2 rates, 0.0625 pounds/acre (lb/ac) and 0.125 lb/ac. The composition of the herbicides and polynucleotides also contain one percent Silwet L77.

*A. palmeri* (R-22) were treated with the compositions listed in Table 6 when they were between 4-8 centimeters tall and had 6-12 leaves, there were 6 replications in the experiment. The effect of the composition was measured as percent control relative to an untreated control 14 days after treatment. Table 6 shows that the composition containing the polynucleotides and glyphosate had enhanced herbicidal activity when 2,4-D was included in the composition as demonstrated by the reduced rate needed to provide the same level of percent control as twice the amount.

**Table 6. Addition of 2,4-D to glyphosate and trigger polynucleotides**

| **Treatment Description** | **% Control (mean)** |
|---|---|
| Roundup WeatherMAX (1.5/A) | 51.7 |
| 2,4-D (0.0625lb/A) | 60 |
| 2,4-D (0.125lb/A) | 80.8 |
| RU Wmax (1.5lb/A) + 2,4-D (0.0625lb/A) | 57.5 |
| RU Wmax (1.5lb/A) + 2,4-D (0.125lb/A) | 77.5 |
| 4002 + RU Wmax (1.5lb/A) + 2,4-D (0.0625lb/A) | 80.8 |
| 4002 + RU Wmax (1.5lb/A) + 2,4-D (0.125lb/A) | 88.3 |

### Example 8. A method to control weeds in a field.

A method to control weeds in a field comprises the use of trigger polynucleotides that can modulate the expression of an EPSPS gene in one or more target weed plant species. Example 5 showed that a weed control composition comprising multiple herbicides and multiple polynucleotides can be used in a field environment to control *A. palmeri* plant growth. An analysis of EPSPS gene sequences from 20 plant species provided a collection of 21-mer polynucleotides (SEQ ID NO:3223-3542) that can be used in compositions to affect the growth or develop or sensitivity to glyphosate herbicide to control multiple weed species in a field. A composition containing 1 or 2 or 3 or 4 or more of the polynucleotides of SEQ ID NO:3223-3542 would enable broad activity of the composition against the multiple weed species or variant populations that occur in a field environment.

The method includes creating an agricultural chemical composition that comprises components that include at least one polynucleotide of SEQ ID NO:3223-3542 or any other effective gene expression modulating polynucleotide essentially identical or essentially complementary to SEQ ID NO: 1-120 or fragment thereof, a transfer agent that mobilizes the polynucleotide into a plant cell and a glyphosate containing herbicide and optionally a polynucleotide that modulates the expression of an essential gene and optionally a herbicide that has a different mode of action relative to glyphosate. The polynucleotide of the composition includes a dsRNA, ssDNA or dsDNA or a combination thereof. A composition containing a polynucleotide can have a use rate of about 1 to 30 grams or more per acre depending on the size of the polynucleotide and the number of polynucleotides in the composition. The composition may include one or more additional herbicides as needed to provide effective multi-species weed control. For example, a composition comprising an EPSPS gene trigger oligonucleotide, the composition further including a co-herbicide but not limited to acetochlor, acifluorfen, acifluorfen-sodium, aclonifen, acrolein, alachlor, alloxydim, allyl alcohol, ametryn, amicarbazone, amidosulfuron, aminopyralid, amitrole, ammonium sulfamate, anilofos, asulam, atraton, atrazine, azimsulfuron, BCPC, beflubutamid, benazolin, benfluralin, benfuresate, bensulfuron, bensulfuron-methyl, bensulide, bentazone, benzfendizone, benzobicyclon, benzofenap, bifenox, bilanafos, bispyribac, bispyribac-sodium, borax, bromacil, bromobutide, bromoxynil, butachlor, butafenacil, butamifos, butralin, butroxydim, butylate, cacodylic acid, calcium chlorate, cafenstrole, carbetamide, carfentrazone, carfentrazone-ethyl, CDEA, CEPC, chlorflurenol, chlorflurenol-methyl, chloridazon, chlorimuron, chlorimuron-ethyl, chloroacetic acid, chlorotoluron, chlorpropham, chlorsulfuron, chlorthal, chlorthaldimethyl, cinidon-ethyl, cinmethylin, cinosulfuron, cisanilide, clethodim, clodinafop, clodinafop-propargyl, clomazone, clomeprop, clopyralid, cloransulam, cloransulammethyl, CMA, 4-CPB, CPMF, 4-CPP, CPPC, cresol, cumyluron, cyanamide, cyanazine, cycloate, cyclosulfamuron, cycloxydim, cyhalofop, cyhalofop-butyl, 2,4-D, 3,4-DA, daimuron, dalapon, dazomet, 2,4-DB, 3,4-DB, 2,4-DEB, desmedipham, dicamba, dichlobenil, ortho-dichlorobenzene, para-dichlorobenzene, dichlorprop, dichlorprop-P, diclofop, diclofop-methyl, diclosulam, difenzoquat, difenzoquat metilsulfate, diflufenican, diflufenzopyr, dimefuron, dimepiperate, dimethachlor, dimethametryn, dimethenamid, dimethenamid-P, dimethipin, dimethylarsinic acid, dinitramine, dinoterb, diphenamid, diquat, diquat dibromide, dithiopyr, diuron, DNOC, 3,4-DP, DSMA, EBEP, endothal, EPTC, esprocarb, ethalfluralin, ethametsulfuron, ethametsulfuron-methyl, ethofumesate, ethoxyfen, ethoxysulfuron, etobenzanid, fenoxaprop-P, fenoxaprop-P-ethyl, fentrazamide, ferrous sulfate, flamprop-M, flazasulfuron, florasulam, fluazifop, fluazifop-butyl, fluazifop-P, fluazifop-P-butyl, flucarbazone, flucarbazone-sodium, flucetosulfuron, fluchloralin, flufenacet, flufenpyr, flufenpyr-ethyl, flumetsulam, flumiclorac, flumicloracpentyl, flumioxazin, fluometuron, fluoroglycofen, fluoroglycofen-ethyl, flupropanate, flupyrsulfuron, flupyrsulfuron-methyl-sodium, flurenol, fluridone, fluorochloridone, fluoroxypyr, flurtamone, fluthiacet, fluthiacet-methyl, fomesafen, foramsulfuron, fosamine, glufosinate, glufosinate-ammonium, halosulfuron, halosulfuron-methyl, haloxyfop, haloxyfop-P, HC-252, hexazinone, imazamethabenz, imazamethabenz-methyl, imazamox, imazapic, imazapyr, imazaquin, imazethapyr, imazosulfuron, indanofan, iodomethane, iodosulfuron, iodosulfuron-methyl-sodium, ioxynil, isoproturon, isouron, isoxaben, isoxachlortole, isoxaflutole, karbutilate, lactofen, lenacil, linuron, MAA, MAMA, MCPA, MCPA-thioethyl, MCPB, mecoprop, mecoprop-P, mefenacet, mefluidide, mesosulfuron, mesosulfuron-methyl, mesotrione, metam, metamifop, metamitron, metazachlor, methabenzthiazuron, methylarsonic acid, methyldymron, methyl isothiocyanate, metobenzuron, metolachlor, S-metolachlor, metosulam, metoxuron, metribuzin, metsulfuron, metsulfuron-methyl, MK-66, molinate, monolinuron, MSMA, naproanilide, napropamide, naptalam, neburon, nicosulfuron, nonanoic acid, norflurazon, oleic acid (fatty acids), orbencarb, orthosulfamuron, oryzalin, oxadiargyl, oxadiazon, oxasulfuron, oxaziclomefone, oxyfluorfen, paraquat, paraquat dichloride, pebulate, pendimethalin, penoxsulam, pentachlorophenol, pentanochlor, pentoxazone, pethoxamid, petrolium oils, phenmedipham, phenmedipham-ethyl, picloram, picolinafen, pinoxaden, piperophos, potassium arsenite, potassium azide, pretilachlor, primisulfuron, primisulfuron-methyl, prodiamine, profluazol, profoxydim, prometon, prometryn, propachlor, propanil, propaquizafop, propazine, propham, propisochlor, propoxycarbazone, propoxycarbazone-sodium, propyzamide, prosulfocarb, prosulfuron, pyraclonil, pyraflufen, pyraflufen-ethyl, pyrazolynate, pyrazosulfuron, pyrazosulfuronethyl, pyrazoxyfen, pyribenzoxim, pyributicarb, pyridafol, pyridate, pyriftalid, pyriminobac, pyriminobac-methyl, pyrimisulfan, pyrithiobac, pyrithiobac-sodium, quinclorac, quinmerac, quinoclamine, quizalofop, quizalofop-P, rimsulfuron, sethoxydim, siduron, simazine, simetryn, SMA, sodium arsenite, sodium azide, sodium chlorate, sulcotrione, sulfentrazone, sulfometuron, sulfometuron-methyl, sulfosate, sulfosulfuron, sulfuric acid, tar oils, 2,3,6-TBA, TCA, TCA-sodium, tebuthiuron, tepraloxydim, terbacil, terbumeton, terbuthylazine, terbutryn, thenylchlor, thiazopyr, thifensulfuron, thifensulfuron-methyl, thiobencarb, tiocarbazil, topramezone, tralkoxydim, tri-allate, triasulfuron, triaziflam, tribenuron, tribenuron-methyl, tricamba, triclopyr, trietazine, trifloxysulfuron, trifloxysulfuron-sodium, trifluralin, triflusulfuron, triflusulfuron-methyl, trihydroxytriazine, tritosulfuron, [3-[2-chloro-4-fluoro-5-(-methyl-6-trifluoromethyl-2,4-dioxo-,2,3,4-t- etrahydropyrimidin-3-yl)phenoxy]-2-pyridyloxy]acetic acid ethyl ester (CAS RN 353292-3-6), 4-[(4,5-dihydro-3-methoxy-4-methyl-5-oxo)-H-,2,4-triazol--ylcarbonylsulfamoyl]-5-methylthiophene-3-carboxylic acid (BAY636), BAY747 (CAS RN 33504-84-2), topramezone (CAS RN 2063-68-8), 4-hydroxy-3-[[2-[(2-methoxyethoxy)methyl]-6-(trifluoro-methyl)-3-pyridi- nyl]carbonyl]-bicyclo[3.2.]oct-3-en-2-one (CAS RN 35200-68-5), and 4-hydroxy-3-[[2-(3-methoxypropyl)-6-(difluoromethyl)-3-pyridinyl]carbon-yl]-bicyclo[3.2.]oct-3-en-2-one.

A field of crop plants in need of weed plant control is treated by spray application of the composition. The composition can be provided as a tank mix, a sequential treatment of components (generally the polynucleotide followed by the herbicide), a simultaneous treatment or mixing of one or more of the components of the composition from separate containers. Treatment of the field can occur as often as needed to provide weed control and the components of the composition can be adjusted to target specific weed species or weed families.

### Example 9. Herbicidal Compositions comprising pesticidal agents

A method of controlling weeds and plant pest and pathogens in a field of glyphosate tolerant crop plants is provided, wherein the method comprises applying a composition comprising an EPSPS trigger oligonucleotide, a glyphosate composition and an admixture of a pest control agent. For example, the admixture comprises insecticides, fungicides, nematocides, bactericides, acaricides, growth regulators, chemosterilants, semiochemicals, repellents, attractants, pheromones, feeding stimulants or other biologically active compounds or biological agents, such as, microorganisms.

For example, the admixture comprises a fungicide compound for use on a glyphosate tolerant crop plant to prevent or control plant disease caused by a plant fungal pathogen, The fungicide compound of the admixture may be a systemic or contact fungicide or mixtures of each. More particularly the fungicide compound includes, but is not limited to members of the chemical groups strobilurins, triazoles, chloronitriles, carboxamides and mixtures thereof. The composition may additional have an admixture comprises an insecticidal compound or agent.

The EPSPS trigger oligonucleotides and WeatherMAX® (WMAX) tank mixes with fungicides, insecticides or both are tested for use in soybean. Soybean rust is a significant problem disease in South America and serious concern in the U.S. Testing is conducted to develop a method for use of mixtures of the WMAX formulation and various commercially available fungicides for weed control and soy rust control. The field plots are planted with Roundup Ready® soybeans. All plots receive a post plant application of the EPSPS trigger +WMAX about 3 weeks after planting. The mixtures of trigger +WMAX or trigger +WMAX + fungicide + insecticides are used to treat the plots at the R1 stage of soybean development (first flowering) of treatment. Data is taken for percent weed control at 7 and 21 days after R1 treatment, soybean safety (percent necrosis, chlorosis, growth rate): 5 days after treatment, disease rating, and soybean yield (bushels/Acre). These mixtures and treatments are designed to provide simultaneous weed and pest control of soybean, such as fungal pest control, for example, soybean rust disease; and insect pest control, for example, aphids, armyworms, loopers, beetles, stinkbugs, and leaf hoppers.

Agricultural chemicals are provided in containers suitable for safe storage, transportation and distribution, stability of the chemical compositions, mixing with solvents and instructions for use. A container of a mixture of a trigger oligonucleotide + glyphosate + fungicide compound, or a mixture of a trigger oligonucleotide + glyphosate compound and an insecticide compound, or a trigger oligonucleotide + a glyphosate compound and a fungicide compound and an insecticide compound (for example, lambda-cyhalothrin, Warrier®). The container may further provide instructions on the effective use of the mixture. Containers can be of any material that is suitable for the storage of the chemical mixture. Containers can be of any material that is suitable for the shipment of the chemical mixture. The material can be of cardboard, plastic, metal, or a composite of these materials. The container can have a volume of 0.5 liter, 1 liter, 2 liter, 3-5 liter, 5-10 liter, 10-20 liter, 20-50 liter or more depending upon the need. A tank mix of a trigger oligonucleotide + glyphosate compound and a fungicide compound is provided, methods of application to the crop to achieve an effective dose of each compound are known to those skilled in the art and can be refined and further developed depending on the crop, weather conditions, and application equipment used.

Insecticides, fungicides, nematocides, bactericides, acaricides, growth regulators, chemosterilants, semiochemicals, repellents, attractants, pheromones, feeding stimulants or other biologically active compounds can be added to the trigger oligonucleotide to form a multi-component pesticide giving an even broader spectrum of agricultural protection. Examples of such agricultural protectants with which compounds of this invention can be formulated are: insecticides such as abamectin, acephate, azinphos-methyl, bifenthrin, buprofezin, carbofuran, chlorfenapyr, chlorpyrifos, chlorpyrifos-methyl, cyfluthrin, betacyfluthrin, cyhalothrin, lambda-cyhalothrin, deltamethrin, diafenthiuron, diazinon, diflubenzuron, dimethoate, esfenvalerate, fenoxycarb, fenpropathrin, fenvalerate, fipronil, flucythrinate, tau-fluvalinate, fonophos, imidacloprid, isofenphos, malathion, metaldehyde, methamidophos, methidathion, methomyl, methoprene, methoxychlor, methyl 7-chloro-2,5-dihydro-2-[[N-(methoxycarbonyl)-N-[4-(trifluoromethoxy)phenyl ]amino]carbonyl]indeno[1,2-e][1,3,4]oxadiazine-4a(3H)-carboxylate (DPX-JW062), monocrotophos, oxamyl, parathion, parathion-methyl, permethrin, phorate, phosalone, phosmet, phosphamidon, pirimicarb, profenofos, rotenone, sulprofos, tebufenozide, tefluthrin, terbufos, tetrachlorvinphos, thiodicarb, tralomethrin, trichlorfon and triflumuron; most preferably a glyphosate compound is formulated with a fungicide compound or combinations of fungicides, such as azoxystrobin, benomyl, blasticidin-S, Bordeaux mixture (tribasic copper sulfate), bromuconazole, captafol, captan, carbendazim, chloroneb, chlorothalonil, copper oxychloride, copper salts, cymoxanil, cyproconazole, cyprodinil (CGA 219417), diclomezine, dicloran, difenoconazole, dimethomorph, diniconazole, diniconazole-M, dodine, edifenphos, epoxiconazole (BAS 480F), famoxadone, fenarimol, fenbuconazole, fenpiclonil, fenpropidin, fenpropimorph, fluazinam, fluquinconazole, flusilazole, flutolanil, flutriafol, folpet, fosetyl-aluminum, furalaxyl, hexaconazole, ipconazole, iprobenfos, iprodione, isoprothiolane, kasugamycin, kresoxim-methyl, mancozeb, maneb, mepronil, metalaxyl, metconazole, S-methyl 7-benzothiazolecarbothioate (CGA 245704), myclobutanil, neo-asozin (ferric methanearsonate), oxadixyl, penconazole, pencycuron, probenazole, prochloraz, propiconazole, pyrifenox, pyroquilon, quinoxyfen, spiroxamine (KWG4168), sulfur, tebuconazole, tetraconazole, thiabendazole, thiophanate-methyl, thiram, triadimefon, triadimenol, tricyclazole, trifloxystrobin, triticonazole, validamycin and vinclozolin; combinations of fungicides are common for example, cyproconazole and azoxystrobin, difenoconazole, and metalaxyl-M, fludioxonil and metalaxyl-M, mancozeb and metalaxyl-M, copper hydroxide and metalaxyl-M, cyprodinil and fludioxonil, cyproconazole and propiconazole; commercially available fungicide formulations for control of Asian soybean rust disease include, but are not limited to Quadris® (Syngenta Corp), Bravo® (Syngenta Corp), Echo 720® (Sipcam Agro Inc), Headline® 2.09EC (BASF Corp), Tilt® 3.6EC (Syngenta Corp), PropiMax™ 3.6EC (Dow AgroSciences), Bumper® 41.8EC (MakhteshimAgan), Folicur® 3.6F (Bayer CropScience), Laredo® 25EC (Dow AgroSciences), Laredo™ 25EW (Dow AgroSciences), Stratego® 2.08F (Bayer Corp), Domark™ 125SL (Sipcam Agro USA), and Pristine®38%WDG (BASF Corp) these can be combined with glyphosate compositions as described in the present invention to provide enhanced protection from soybean rust disease; nematocides such as aldoxycarb and fenamiphos; bactericides such as streptomycin; acaricides such as amitraz, chinomethionat, chlorobenzilate, cyhexatin, dicofol, dienochlor, etoxazole, fenazaquin, fenbutatin oxide, fenpropathrin, fenpyroximate, hexythiazox, propargite, pyridaben and tebufenpyrad; and biological agents such as *Bacillus thuringiensis, Bacillus thuringiensis* delta endotoxin, baculovirus, and entomopathogenic bacteria, virus and fungi.

**Table 1. EPSPS gene polynucleotide sequences**

| SEQ ID NO | SPECIES | TYPE | LENGTH |
|---|---|---|---|
| 1 | Agrobacterium tumefaciens | cDNA | 1362 |
| 2 | Abutilon theophrasti | cDNA | 1622 |
| 3 | Amaranthus graecizans | cDNA | 958 |
| 4 | Amaranthus graecizans | cDNA | 490 |
| 5 | Amaranthus hybridus | cDNA | 1682 |
| 6 | Amaranthus lividus | cDNA | 843 |
| 7 | Amaranthus palmeri | cDNAContig | 1554 |
| 8 | Amaranthus palmeri | Genomic | 18729 |
| 9 | Amaranthus palmeri | Genomic | 668 |
| 10 | Amaranthus palmeri | Genomic | 13434 |
| 11 | Amaranthus palmeri | Genomic | 38 |
| 12 | Amaranthus palmeri | cDNA | 1911 |
| 13 | Amaranthus rudis | cDNAContig | 1554 |
| 14 | Amaranthus rudis | Genomic | 2425 |
| 15 | Amaranthus rudis | Genomic | 2013 |
| 16 | Amaranthus rudis | Genomic | 1530 |
| 17 | Amaranthus rudis | Genomic | 1145 |
| 18 | Amaranthus rudis | Genomic | 703 |
| 19 | Amaranthus rudis | Genomic | 231 |
| 20 | Amaranthus rudis | Genomic | 208 |
| 21 | Amaranthus rudis | Genomic | 94 |
| 22 | Amaranthus rudis | Genomic | 40 |
| 23 | Amaranthus rudis | Genomic | 3681 |
| 24 | Amaranthus rudis | Genomic | 589 |
| 25 | Amaranthus rudis | Genomic | 479 |
| 26 | Amaranthus rudis | Genomic | 473 |
| 27 | Amaranthus rudis | Genomic | 417 |
| 28 | Amaranthus rudis | Genomic | 224 |
| 29 | Amaranthus rudis | cDNA | 2086 |
| 30 | Amaranthus rudis | cDNA | 1960 |
| 31 | Amaranthus spinosus | cDNA | 939 |
| 32 | Amaranthus spinosus | cDNA | 381 |
| 33 | Amaranthus thunbergii | cDNA | 966 |
| 34 | Amaranthus thunbergii | cDNA | 484 |
| 35 | Amaranthus viridis | cDNA | 2329 |
| 36 | Amaranthus viridis | cDNA | 1746 |
| 37 | Ambrosia artemisiifolia | Genomic | 1340 |
| 38 | Ambrosia artemisiifolia | Genomic | 1264 |
| 39 | Ambrosia artemisiifolia | Genomic | 910 |
| 40 | Ambrosia artemisiifolia | Genomic | 732 |
| 41 | Ambrosia artemisiifolia | Genomic | 278 |
| 42 | Ambrosia trifida | cDNAContig | 1503 |
| 43 | Ambrosia trifida | Genomic | 1465 |
| 44 | Ambrosia trifida | Genomic | 1022 |
| 45 | Ambrosia trifida | Genomic | 697 |
| 46 | Ambrosia trifida | Genomic | 439 |
| 47 | Ambrosia trifida | Genomic | 436 |
| 48 | Ambrosia trifida | Genomic | 404 |
| 49 | Ambrosia trifida | Genomic | 1209 |
| 50 | Ambrosia trifida | Genomic | 984 |
| 51 | Ambrosia trifida | Genomic | 980 |
| 52 | Ambrosia trifida | Genomic | 429 |
| 53 | Ambrosia trifida | Genomic | 234 |
| 54 | Ambrosia trifida | Genomic | 219 |
| 55 | Ambrosia trifida | Genomic | 26 |
| 56 | Ambrosia trifida | cDNA | 1721 |
| 57 | Ambrosia trifida | cDNA | 1689 |
| 58 | Chenopodium album | cDNA | 1432 |
| 59 | Conyza canadensis | Genomic | 15055 |
| 60 | Conyza canadensis | Genomic | 12729 |
| 61 | Conyza canadensis | Genomic | 2833 |
| 62 | Conyza canadensis | Genomic | 15010 |
| 63 | Conyza canadensis | Genomic | 12222 |
| 64 | Conyza canadensis | cDNA | 1882 |
| 65 | Conyza canadensis | cDNA | 1800 |
| 66 | Conyza canadensis | cDNA | 1730 |
| 67 | Conyza canadensis | Genomic | 7954 |
| 68 | Conyza canadensis | Genomic | 6988 |
| 69 | Euphorbia heterophylla | cDNAContig | 1563 |
| 70 | Euphorbia heterophylla | Genomic | 9336 |
| 71 | Euphorbia heterophylla | Genomic | 6002 |
| 72 | Euphorbia heterophylla | Genomic | 5555 |
| 73 | Euphorbia heterophylla | Genomic | 4647 |
| 74 | Euphorbia heterophylla | Genomic | 378 |
| 75 | Euphorbia heterophylla | Genomic | 220 |
| 76 | Euphorbia heterophylla | Genomic | 4459 |
| 77 | Euphorbia heterophylla | Genomic | 1339 |
| 78 | Euphorbia heterophylla | cDNA | 1668 |
| 79 | Euphorbia heterophylla | cDNA | 783 |
| 80 | Euphorbia heterophylla | Genomic | 2185 |
| 81 | Euphorbia heterophylla | Genomic | 1702 |
| 82 | Euphorbia heterophylla | Genomic | 1400 |
| 83 | Euphorbia heterophylla | Genomic | 584 |
| 84 | Commelina diffusa | cDNA | 1250 |
| 85 | Commelina diffusa | Genomic | 9352 |
| 86 | Commelina diffusa | Genomic | 6205 |
| 87 | Commelina diffusa | Genomic | 818 |
| 88 | Commelina diffusa | Genomic | 127 |
| 89 | Digitaria sanguinalis | cDNA | 783 |
| 90 | Digitaria sanguinalis | cDNA | 679 |
| 91 | Digitaria sanguinalis | cDNA | 638 |
| 92 | Digitaria sanguinalis | cDNA | 605 |
| 93 | Digitaria sanguinalis | cDNA | 605 |
| 94 | Digitaria sanguinalis | cDNA | 510 |
| 95 | Digitaria sanguinalis | cDNA | 510 |
| 96 | Digitaria sanguinalis | cDNA | 477 |
| 97 | Kochia scoparia | cDNAContig | 1548 |
| 98 | Kochia scoparia | Genomic | 7037 |
| 99 | Kochia scoparia | Genomic | 5741 |
| 100 | Kochia scoparia | Genomic | 4546 |
| 101 | Kochia scoparia | Genomic | 57 |
| 102 | Kochia scoparia | cDNAContig | 1548 |
| 103 | Kochia scoparia | Genomic | 5426 |
| 104 | Kochia scoparia | Genomic | 2430 |
| 105 | Kochia scoparia | Genomic | 4880 |
| 106 | Lolium multiflorum | Genomic | 6967 |
| 107 | Lolium multiflorum | Genomic | 1093 |
| 108 | Lolium multiflorum | Genomic | 983 |
| 109 | Lolium multiflorum | Genomic | 591 |
| 110 | Lolium multiflorum | Genomic | 514 |
| 111 | Lolium multiflorum | Genomic | 460 |
| 112 | Lolium multiflorum | Genomic | 1377 |
| 113 | Lolium multiflorum | Genomic | 1107 |
| 114 | Lolium multiflorum | Genomic | 480 |
| 115 | Lolium multiflorum | Genomic | 318 |
| 116 | Lolium multiflorum | cDNA | 1284 |
| 117 | Lolium rigidium | Genomic | 302 |
| 118 | Sorghum halepense | cDNA | 608 |
| 119 | Lolium rigidium | Genomic | 647 |
| 120 | Lolium rigidium | Genomic | 4472 |

## Claims

1. A method of plant control comprising: topically applying to a surface of a plant a composition comprising a double-stranded RNA (dsRNA) polynucleotide and a transfer agent, wherein said dsRNA polynucleotide is identical or complementary to an RNA sequence of a 5-enolpyruvylshikimate-3-phosphate synthase (EPSPS) gene sequence selected from the group consisting of SEQ ID NOs: 11, 18, 19, 21, 22, 53-55, 61, 72, 74, 75, 84, 88, 99, 101, 110, 115, 119, and an at least 18 contiguous nucleotides polynucleotide fragment thereof, wherein said transfer agent is an organosilicone surfactant composition or an organosilicone compound contained therein and conditions said surface of said plant for permeation by said dsRNA polynucleotide, and whereby said plant's growth, development, or reproductive ability is reduced or said plant is more sensitive to an EPSPS inhibitor herbicide relative, to an untreated plant.

2. The method as claimed in claim 1, wherein
(i) said plant is selected from the group consisting of *Amaranthus palmeri, Amaranthus rudis, Amaranthus graecizans, Amaranthus hybridus, Amaranthus lividus, Amaranthus spinosus, Amaranthus thunbergii, Amaranthus viridis, Lolium multiflorum, Lolium rigidum, Ambrosia artemisiifolia, Ambrosia trifida, Euphorbia heterophylla, Kochia scoparia, Abutilon theophrasti, Sorghum halepense, Chenopodium album, Commelina diffusa, Conyza canadensis,* and *Digitaria sanguinalis;* or
(ii) said composition further comprises
(a) said EPSPS inhibitor herbicide;
(b) one or more herbicides different from said EPSPS inhibitor herbicide;
(c) an auxin-like herbicide; or
(d) 3,6-dichloro-2-methoxybenzoic acid or 2,4-dichlorophenoxyacetic acid.

3. The method as claimed in claim 1, wherein said dsRNA polynucleotide is at least 21 contiguous nucleotides in length or said composition comprises any combination of two or more of said dsRNA polynucleotides.

4. A composition for topical application to a surface of a plant comprising a dsRNA polynucleotide and a transfer agent, wherein said dsRNA polynucleotide is identical or complementary to an RNA sequence of an EPSPS gene sequence selected from the group consisting of SEQ ID NOs: 11, 18, 19, 21, 22, 53-55, 61, 72, 74, 75, 84, 88, 99, 101, 110, 115, 119, and an at least 18 contiguous nucleotides polynucleotide fragment thereof, wherein said transfer agent is an organosilicone surfactant composition or an organosilicone compound contained therein and conditions said surface of said plant for permeation by said dsRNA polynucleotide, and whereby said plant treated with said composition has its growth, development, or reproductive ability suppressed or delayed or said plant is more sensitive to an EPSPS inhibitor herbicide as a result of said dsRNA polynucleotide containing composition, relative to an untreated plant.

5. The composition of claim 4, wherein
(i) said dsRNA polynucleotide is at least 21 contiguous nucleotides in length;
(ii) said dsRNA polynucleotide is selected from the group consisting of SEQ ID NOs: 121-126, 137-140, 143-148, 213, 214, 231-240, 259-276, 281-308, 313-332, 345-356, 367-380, 383-442, 449-460, 479-496, 501-528, 533-550, 565-576, 591-600, 603-630, 635-642, 659-666, 687, 688, 709-718, 751, 752, 755-760, 765, 766, 771-784, 799-804, 811-818, 857-872, 893-898, 901, 902, 907-912, 915, 916, 921-930, 933, 934, 943-950, 955-958, 971, 972, 981-984, 989, 990, 993-1002, 1007, 1008, 1017, 1018, 1021, 1022, 1025-1032, 1035, 1036, 1041-1048, 1051-1056, 1059, 1060, 1071-1104, 1107-1154, 1159-1186, 1189-1290, 1293-1304, 1307-1322, 1325-1336, 1341-1344, 1347-1350, 1353-1472, 1477-1480, 1489-1492, 1497-1500, 1507-1522, 1527-1540, 1543-1546, 1551-1564, 1567-1584, 1587-1604, 1607-1678, 1681-1694, 1697-1702, 1707-1718, 1723-1738, 1741-1756, 1763-1792, 1797-1800, 1809-1824, 1827-1838, 1841-1856, 1859-1870, 1875-1878, 1881-1884, 1887-1932, 1935-1948, 1951-1956, 1961-1972, 1977-2000, 2003-2014, 2017-2032, 2035-2046, 2051-2054, 2057-2060, 2063-2146, 2149, 2150, 2161, 2162, 2167, 2168, 2187-2194, 2197, 2198, 2211-2256, 2259-2264, 2267, 2268, 2271-2276, 2283-2308, 2311-2388, 2391, 2392, 2395-2402, 2405, 2406, 2409, 2410, 2413, 2414, 2417-2426, 2429-2464, 2467, 2468, 2471-2476, 2483-2500, 2503-2514, 2517-2532, 2539-2550, 2557-2602, 2605-2608, 2611-2634, 2637-2640, 2643-2646, 2649, 2650, 2663-2730, 2735-2738, 2745-2774, 2779-2796, 2799-2820, 2823-2842, 2845-2854, 2857, 2858, 2863-2870, 2873-2880, 2887, 2888, 2891-2896, 2901-2904, 2911-2920, 2927, 2928, 2931-2936, 2941-2944, 2951-2958, 2963-3000, 3019, 3020, 3043-3054, 3067-3074, 3085-3146, 3151, 3152, 3155-3158, 3161-3174, 3187-3194, 3203, 3204, and 3207-3216;
(iii) said dsRNA polynucleotide is selected from the group consisting of SEQ ID NOs: 3235, 3258, 3260, 3268, 3280, 3283, 3289, 3307, 3308, 3313, 3320, 3338, 3344, 3345, 3351, 3370, 3397, 3399, 3411, 3431, 3438, 3449, 3452, 3455, 3467, 3472, 3479, 3485, 3492, 3517, 3521, 3524, 3530, 3533, 3538, and 3542; or
(iv) said composition further comprises
(a) said EPSPS inhibitor herbicide;
(b) glyphosate;
(c) a co-herbicide;
(d) an auxin-like herbicide; or
(e) 3,6-dichloro-2-methoxybenzoic acid or 2,4-dichlorophenoxyacetic acid.

6. A method of reducing expression of an EPSPS gene in a plant comprising: topically applying to a surface of a plant a composition comprising a dsRNA polynucleotide and a transfer agent, wherein said dsRNA polynucleotide is identical or complementary to an RNA sequence of an EPSPS gene sequence selected from the group consisting of SEQ ID NOs: 11, 18, 19, 21, 22, 53-55, 61, 72, 74, 75, 84, 88, 99, 101, 110, 115, 119, and an at least 18 contiguous nucleotides polynucleotide fragment thereof, wherein said transfer agent is an organosilicone surfactant composition or an organosilicone compound contained therein and conditions the surface of said plant for permeation by said dsRNA polynucleotide, and whereby said expression of said EPSPS gene is reduced, relative to an untreated plant.

7. The method as claimed in claim 6, wherein
(i) said dsRNA polynucleotide is at least 21 contiguous nucleotides in length.

8. A method of identifying dsRNA polynucleotides useful in modulating EPSPS gene expression when topically applied to a surface of a plant comprising: a) providing a plurality of dsRNA polynucleotides that comprise a region identical or complementary to at least 18 contiguous nucleotides of an RNA sequence of an EPSPS gene sequence selected from the group consisting of SEQ ID NOs: 11, 18, 19, 21, 22, 53-55, 61, 72, 74, 75, 84, 88, 99, 101, 110, 115, and 119; b) topically applying to said surface of said plant a composition comprising one or more of said dsRNA polynucleotides and a transfer agent; and c) analyzing said plant, or a plant extract thereof, for modulation of EPSPS gene expression, wherein said transfer agent is an organosilicone surfactant composition or an organosilicone compound contained therein and conditions the surface of said plant for permeation by said dsRNA polynucleotide, and whereby said plant treated with said composition has its growth, development, or reproductive ability suppressed or delayed or said plant is more sensitive to an EPSPS inhibitor herbicide as a result of said dsRNA polynucleotide containing composition, relative to an untreated plant.

9. The method as claimed in claim 8, wherein
(i) said plant is selected from the group consisting of *Amaranthus palmeri, Amaranthus rudis, Amaranthus graecizans, Amaranthus hybridus, Amaranthus lividus, Amaranthus spinosus, Amaranthus thunbergii, Amaranthus viridis, Lolium multiflorum, Lolium rigidum, Ambrosia artemisiifolia, Ambrosia trifida, Euphorbia heterophylla, Kochia scoparia, Abutilon theophrasti, Sorghum halepense, Chenopodium album, Commelina diffusa, Conyza canadensis,* and *Digitaria sanguinalis;* or
(ii) said EPSPS gene expression is reduced relative to a plant not treated with said composition.

10. An agricultural chemical composition for topical application to a surface of a plant comprising an admixture of a dsRNA polynucleotide, a transfer agent, glyphosate, and a co-herbicide, wherein said dsRNA polynucleotide is identical or complementary to an RNA sequence of an EPSPS gene sequence selected from the group consisting of SEQ ID NOs: 11, 18, 19, 21, 22, 53-55, 61, 72, 74, 75, 84, 88, 99, 101, 110, 115, 119, and an at least 18 contiguous nucleotides polynucleotide fragment thereof, wherein said transfer agent is an organosilicone surfactant composition or an organosilicone compound contained therein and conditions the surface of said plant for permeation by said dsRNA polynucleotide and whereby said plant treated with said composition has its growth, development, or reproductive ability suppressed or delayed or said plant is more sensitive to an EPSPS inhibitor herbicide as a result of said dsRNA polynucleotide containing composition, relative to an untreated plant.

11. The agricultural chemical composition of claim 10, wherein said co-herbicide is selected from the group consisting of amide herbicides, arsenical herbicides, benzothiazole herbicides, benzoylcyclohexanedione herbicides, benzofuranyl alkylsulfonate herbicides, carbamate herbicides, cyclohexene oxime herbicides, cyclopropylisoxazole herbicides, dicarboximide herbicides, dinitroaniline herbicides, dinitrophenol herbicides, diphenyl ether herbicides, dithiocarbamate herbicides, halogenated aliphatic herbicides, imidazolinone herbicides, inorganic herbicides, nitrile herbicides, organophosphorus herbicides, oxidiazolone herbicides, oxazole herbicides, phenoxy herbicides, phenylenediamine herbicides, pyrazole herbicides, pyridazine herbicides, pyridazinone herbicides, pyridine herbicides, pyrimidinediamine herbicides, pyrimidinyloxybenzylamine herbicides, quaternary ammonium herbicides, thiocarbamate herbicides, thiocarbonate herbicides, thiourea herbicides, triazine herbicides, triazinone herbicides, triazole herbicides, triazolone herbicides, triazolopyrimidine herbicides, uracil herbicides and urea herbicides.

12. An agricultural chemical composition for topical application to a surface of a plant comprising an admixture of a dsRNA polynucleotide, a transfer agent glyphosate, and a pesticide, wherein said dsRNA polynucleotide is identical or complementary to an RNA sequence of an EPSPS gene sequence selected from the group consisting of SEQ ID NOs: 11, 18, 19, 21, 22, 53-55, 61, 72, 74, 75, 84, 88, 99, 101, 110, 115, 119, and an at least 18 contiguous nucleotides polynucleotide fragment thereof, wherein said transfer agent is an organosilicone surfactant composition or an organosilicone compound contained therein and conditions the surface of said plant for permeation by said dsRNA polynucleotide and whereby said plant treated with said composition has its growth, development, or reproductive ability suppressed or delayed or said plant is more sensitive to an EPSPS inhibitor herbicide as a result of said dsRNA polynucleotide containing composition, relative to an untreated plant.

13. The agricultural chemical composition of claim 12, wherein said pesticide is selected from the group consisting of insecticides, fungicides, nematicides, bactericides, acaricides, growth regulators, chemosterilants, semiochemicals, repellents, attractants, pheromones, feeding stimulants and biopesticides.

## Patentansprüche

1. Verfahren zur Kontrolle von Pflanzen, Schritte umfassend, bei denen man:
eine Zusammensetzung, die ein doppelsträngiges RNA (dsRNA) Polynukleotid und ein Übertragungsmittel umfasst, auf eine Oberfläche einer Pflanze aufbringt, wobei das dsRNA-Polynukleotid identisch oder komplementär zur RNA-Sequenz einer 5-Enolpyruvylshikimate-3-phosphatsynthase (EPSPS) Gensequenz ist, die aus der Gruppe bestehend aus SEQ ID Nummern: 11, 18, 19, 21, 22, 53-55, 61, 72, 74, 75, 84, 88, 99, 101, 110, 115, 119 und einem Polynukleotidfragment davon mit mindestens 18 aufeinanderfolgenden Nukleotiden ausgewählt wurde, wobei das Übertragungsmittel eine siliciumorganische Tensid-Verbindung oder eine darin enthaltene siliciumorganische Verbindung ist, welche die Oberfläche der Pflanze für das Eindringen des dsRNA-Polynukleotids konditioniert, und wobei die Wachstums-, Entwicklungs- oder Fortpflanzungsfähigkeit der Pflanze im Vergleich zu einer unbehandelten Pflanze reduziert wird oder die Pflanze empfindlicher auf ein EPSPS-Inhibitor-Herbizid reagiert.

2. Verfahren gemäß Anspruch 1, bei dem
(i) die Pflanze aus der Gruppe bestehend aus *Amaranthus palmeri, Amaranthus rudis, Amaranthus graecizans, Amaranthus hybridus, Amaranthus lividus, Amaranthus spinosus, Amaranthus thunbergii, Amaranthus viridis, Lolium multiflorum, Lolium rigidum, Ambrosia artemisiifolia, Ambrosia trifida, Euphorbia heterophylla, Kochia scoparia, Abutilon theophrasti, Sorghum halepense, Chenopodium album, Commelina diffusa, Conyza canadensis,* and *Digitaria sanguinalis* ausgewählt wurde; oder
(ii) die Zusammensetzung ferner folgendes umfasst:
(a) ein EPSPS Inhibitor-Herbizid;
(b) ein oder mehrere Herbizide, die sich von dem EPSPS Inhibitor-Herbizid unterscheiden;
(c) ein Auxin-ähnliches Herbizid;
(d) 3,6-Dichlor-2-Methoxybenzoesäure oder 2,4-Dichlorphenoxyessigsäure.

3. Verfahren gemäß Anspruch 1, bei dem das dsRNA-Polynukleotid mindestens 21 aufeinanderfolgende Nukleotide lang ist oder die Zusammensetzung eine beliebige Kombination von zwei oder mehr der dsRNA-Polynukleotide umfasst.

4. Zusammensetzung zur topischen Anwendung auf einer Oberfläche einer Pflanze, die ein doppelsträngiges RNA (dsRNA) Polynukleotid und ein Übertragungsmittel umfasst, wobei das dsRNA-Polynukleotid identisch oder komplementär zur RNA-Sequenz einer EPSPS Gensequenz ist, die aus der Gruppe bestehend aus SEQ ID Nummern: 11, 18, 19, 21, 22, 53-55, 61, 72, 74, 75, 84, 88, 99, 101, 110, 115, 119 und einem Polynukleotidfragment davon mit mindestens 18 aufeinanderfolgenden Nukleotiden ausgewählt wurde, wobei das Übertragungsmittel eine siliciumorganische Tensid-Verbindung oder eine darin enthaltene siliciumorganische Verbindung ist, welche die Oberfläche der Pflanze für das Eindringen des dsRNA-Polynukleotids konditioniert, und wobei die Wachstums-, Entwicklungs- oder Fortpflanzungsfähigkeit der mit der Zusammensetzung behandelten Pflanze im Vergleich zu einer unbehandelten Pflanze unterdrückt oder verzögert wird oder die Pflanze empfindlicher auf ein EPSPS-Inhibitor-Herbizid reagiert.

5. Zusammensetzung gemäß Anspruch 4, bei der:
(i) das dsRNA-Polynukleotid mindestens 21 aufeinanderfolgende Nukleotide lang ist;
(ii) das dsRNA-Polynukleotid aus der Gruppe bestehend aus SEQ ID Nummern: 121-126, 137-140, 143-148, 213, 214, 231-240, 259-276, 281-308, 313-332, 345-356, 367-380, 383-442, 449-460, 479-496, 501-528, 533-550, 565-576, 591-600, 603-630, 635-642, 659-666, 687, 688, 709-718, 751, 752, 755-760, 765, 766, 771-784, 799-804, 811-818, 857-872, 893-898, 901, 902, 907-912, 915, 916, 921-930, 933, 934, 943-950, 955-958, 971, 972, 981-984, 989, 990, 993-1002, 1007, 1008, 1017, 1018, 1021, 1022, 1025-1032, 1035, 1036, 1041-1048, 1051-1056, 1059, 1060, 1071-1104, 1107-1154, 1159-1186, 1189-1290, 1293-1304, 1307-1322, 1325-1336, 1341-1344, 1347-1350, 1353-1472, 1477-1480, 1489-1492, 1497-1500, 1507-1522, 1527-1540, 1543-1546, 1551-1564, 1567-1584, 1587-1604, 1607-1678, 1681-1694, 1697-1702, 1707-1718, 1723-1738, 1741-1756, 1763-1792, 1797-1800, 1809-1824, 1827-1838, 1841-1856, 1859-1870, 1875-1878, 1881-1884, 1887-1932, 1935-1948, 1951-1956, 1961-1972, 1977-2000, 2003-2014, 2017-2032, 2035-2046, 2051-2054, 2057-2060, 2063-2146, 2149, 2150, 2161, 2162, 2167, 2168, 2187-2194, 2197, 2198, 2211-2256, 2259-2264, 2267, 2268, 2271-2276, 2283-2308, 2311-2388, 2391, 2392, 2395-2402, 2405, 2406, 2409, 2410, 2413, 2414, 2417-2426, 2429-2464, 2467, 2468, 2471-2476, 2483-2500, 2503-2514, 2517-2532, 2539-2550, 2557-2602, 2605-2608, 2611-2634, 2637-2640, 2643-2646, 2649, 2650, 2663-2730, 2735-2738, 2745-2774, 2779-2796, 2799-2820, 2823-2842, 2845-2854, 2857, 2858, 2863-2870, 2873-2880, 2887, 2888, 2891-2896, 2901-2904, 2911-2920, 2927, 2928, 2931-2936, 2941-2944, 2951-2958, 2963-3000, 3019, 3020, 3043-3054, 3067-3074, 3085-3146, 3151, 3152, 3155-3158, 3161-3174, 3187-3194, 3203, 3204, und 3207-3216 ausgewählt wurde;
(iii) das dsRNA-Polynukleotid aus der Gruppe bestehend aus SEQ ID Nummern: 3235, 3258, 3260, 3268, 3280, 3283, 3289, 3307, 3308, 3313, 3320, 3338, 3344, 3345, 3351, 3370, 3397, 3399, 3411, 3431, 3438, 3449, 3452, 3455, 3467, 3472, 3479, 3485, 3492, 3517, 3521, 3524, 3530, 3533, 3538, und 3542 ausgewählt wurde; oder
(iv) die Zusammensetzung ferner folgendes umfasst:
(a) ein EPSPS Inhibitor-Herbizid;
(b) Glyphosate;
(c) ein Co-Herbizid;
(d) ein Auxin-ähnliches Herbizid;
(d) 3,6-Dichlor-2-Methoxybenzoesäure oder 2,4-Dichlorphenoxyessigsäure.

6. Verfahren zur Verringerung der Expression eines EPSPS-Gens in einer Pflanze, Schritte umfassend, bei denen man:
eine Zusammensetzung, die ein dsRNA-Polynukleotid und ein Übertragungsmittel umfasst, auf eine Oberfläche einer Pflanze topisch aufbringt, wobei das dsRNA-Polynukleotid identisch oder komplementär zur RNA-Sequenz einer EPSPS Gensequenz ist, die aus der Gruppe bestehend aus SEQ ID Nummern: 11, 18, 19, 21, 22, 53-55, 61, 72, 74, 75, 84, 88, 99, 101, 110, 115, 119 und einem Polynukleotidfragment davon mit mindestens 18 aufeinanderfolgenden Nukleotiden ausgewählt wurde, wobei das Übertragungsmittel eine siliciumorganische Tensid-Verbindung oder eine darin enthaltene siliciumorganische Verbindung ist, welche die Oberfläche der Pflanze für das Eindringen des dsRNA-Polynukleotids konditioniert, und wobei die Expression des EPSPS-Gens in Vergleich zu einer unbehandelten Pflanze verringert wird.

7. Verfahren gemäß Anspruch 6, bei dem:
(i) das dsRNA-Polynukleotid mindestens 21 aufeinanderfolgende Nukleotide lang ist.

8. Verfahren zur Identifizierung von dsRNA-Polynukleotiden, die bei topischer Aufbringung auf eine Oberfläche einer Pflanze für die Modulation der EPSPS-Genexpression verwendet werden können, Schritte umfassend, bei denen man:
a) eine Vielzahl an dsRNA-Polynukleotiden bereitstellt, die einen Bereich umfassen, der identisch oder komplementär zu mindestens 18 aufeinanderfolgenden Nukleotiden einer RNA-Sequenz einer EPSPS-Gensequenz ist, die aus der Gruppe bestehend aus SEQ ID Nummern: 11, 18, 19, 21, 22, 53-55, 61, 72, 74, 75, 84, 88, 99, 101, 110, 115 und 119 ausgewählt wurde;
b) eine Zusammensetzung, die eines oder mehrere der dsRNA-Polynukleotide und ein Übertragungsmittel umfasst, auf die Oberfläche der Pflanze topisch aufbringt; und
c) die Pflanze oder ein Pflanzenextrakt davon auf die Veränderung der EPSPS-Genexpression hin analysiert, wobei das Übertragungsmittel eine siliciumorganische Tensid-Verbindung oder eine darin enthaltene siliciumorganische Verbindung ist, welche die Oberfläche der Pflanze für das Eindringen des dsRNA-Polynukleotids konditioniert, und wobei die dsRNA-Polynukleotide enthaltende Zusammensetzung die Wachstums-, Entwicklungs- oder Fortpflanzungsfähigkeit der Pflanze im Vergleich zu einer unbehandelten Pflanze unterdrückt oder verzögert oder die Pflanze als Ergebnis der Behandlung mit der dsRNA enthaltenden Zusammensetzung empfindlicher auf ein EPSPS-Inhibitor-Herbizid wird.

9. Verfahren gemäß Anspruch 8, bei dem
(i) die Pflanze aus der Gruppe bestehend aus *Amaranthus palmeri, Amaranthus rudis, Amaranthus graecizans, Amaranthus hybridus, Amaranthus lividus, Amaranthus spinosus, Amaranthus thunbergii, Amaranthus viridis, Lolium multiflorum, Lolium rigidum, Ambrosia artemisiifolia, Ambrosia trifida, Euphorbia heterophylla, Kochia scoparia, Abutilon theophrasti, Sorghum halepense, Chenopodium album, Commelina diffusa, Conyza canadensis,* und *Digitaria sanguinalis* ausgewählt wurde; oder
(ii) die Expression des EPSPS-Gens in Vergleich zu einer unbehandelten Pflanze verringert wird.

10. Landwirtschaftliche chemische Zusammensetzung zur topischen Anwendung auf eine Oberfläche einer Pflanze, die eine Mischung aus einem dsRNA-Polynukleotid, einem Übertragungsmittel, Glyphosat und einem Co-Herbizid umfasst, wobei das dsRNA-Polynukleotid identisch oder komplementär zur RNA-Sequenz einer EPSPS Gensequenz ist, die aus der Gruppe bestehend aus SEQ ID Nummern: 11, 18, 19, 21, 22, 53-55, 61, 72, 74, 75, 84, 88, 99, 101, 110, 115, 119 und einem Polynukleotidfragment davon mit mindestens 18 aufeinanderfolgenden Nukleotiden ausgewählt wurde, wobei das Übertragungsmittel eine siliciumorganische Tensid-Verbindung oder eine darin enthaltene siliciumorganische Verbindung ist, welche die Oberfläche der Pflanze für das Eindringen des dsRNA-Polynukleotids konditioniert, und wobei die Wachstums-, Entwicklungs- oder Fortpflanzungsfähigkeit der mit der Zusammensetzung behandelten Pflanze unterdrückt oder verzögert wird oder die Pflanze als Ergebnis der Behandlung mit der dsRNA Polynukleotid enthaltenden Zusammensetzung im Vergleich zu einer unbehandelten Pflanze empfindlicher auf ein EPSPS-Inhibitor-Herbizid reagiert.

11. Landwirtschaftliche chemische Zusammensetzung gemäß Anspruch 10, in der das Co-Herbizid aus Amidherbiziden, Arsenherbiziden, Benzothiazolherbiziden, Benzoylcyclohexandionherbiziden, Benzofuranylalkylsulfonatherbiziden, Cyclohexenoxim Herbiziden, Cyclopropyl-isoxazolherbiziden, Dicarboximidherbiziden, Dinitroanilin-herbiziden, Dinitrophenol-Herbiziden, Diphenylether-Herbiziden, Dithiocarbamat-Herbiziden, halogenierten aliphatischen Herbiziden, Imidazolinon-Herbiziden, anorganische Herbiziden, Nitril-Herbiziden, organischen Phosphor-Herbiziden, Oxadiazolon-Herbiziden, Oxazol-Herbiziden, Phenoxy-Herbiziden, Phenylendiamin-Herbiziden, Pyrazol-Herbiziden, Pyridazin-Herbiziden, Pyridazinon-Herbiziden, Pyridin-Herbiziden, Pyrimidindiamin-Herbiziden, Pyrimidinyloxybenzylamin-Herbiziden, quaternäre Ammonium-Herbiziden, Thiocarbamat-Herbiziden, Thiocarbonat-Herbiziden, Thioharnstoff-Herbiziden, Triazin-Herbiziden, Triazinon Herbiziden, Triazol-Herbiziden, Triazolon-Herbiziden, Triazolopyrimidin-Herbiziden, Uracil-Herbiziden und Harnstoff-Herbiziden ausgewählt wurde.

12. Landwirtschaftliche chemische Zusammensetzung zur topischen Anwendung auf einer Oberfläche einer Pflanze, die eine Mischung aus einem aus einem dsRNA-Polynukleotid, einem Übertragungsmittel und einem Pestizid umfasst, wobei das dsRNA-Polynukleotid identisch oder komplementär zur RNA-Sequenz einer EPSPS Gensequenz ist, die aus der Gruppe bestehend aus SEQ ID Nummern: 11, 18, 19, 21, 22, 53-55, 61, 72, 74, 75, 84, 88, 99, 101, 110, 115, 119 und einem Polynukleotidfragment davon mit mindestens 18 aufeinanderfolgenden Nukleotiden ausgewählt wurde, wobei das Übertragungsmittel eine siliciumorganische Tensid-Verbindung oder eine darin enthaltene siliciumorganische Verbindung ist, welche die Oberfläche der Pflanze für das Eindringen des dsRNA-Polynukleotids konditioniert, und wobei die Wachstums-, Entwicklungs- oder Fortpflanzungsfähigkeit der mit der Zusammensetzung behandelten Pflanze unterdrückt oder verzögert wird oder die Pflanze als Ergebnis der Behandlung mit der dsRNA Polynukleotid enthaltenden Zusammensetzung im Vergleich zu einer unbehandelten Pflanze empfindlicher auf ein EPSPS-Inhibitor-Herbizid reagiert.

13. Landwirtschaftliche chemische Zusammensetzung gemäß Anspruch 12, in der das Pestizid aus der Gruppe bestehend aus Insektiziden, Fungiziden, Nematiziden, Bakteriziden, Akariziden, Wachstumsregulatoren, Chemosterilantien, Semiochemikalien, Abwehrmitteln, Lockstoffen, Pheromonen, Ernährungsstimulanzien und Biopestiziden ausgewählt wurde.

## Revendications

1. Procédé de lutte contre des plantes, comprenant : le fait d'appliquer topiquement sur une surface d'une plante une composition comprenant un polynucléotide ARN double brin (ARNdb) et un agent de transfert, dans lequel ledit polynucléotide ARNdb est identique à ou complémentaire d'une séquence d'ARN d'une séquence de gène de 5-énolpyruvylshikimate-3-phosphate synthase (EPSPS) choisie dans l'ensemble constitué par les séquences SEQ ID NO : 11, 18, 19, 21, 22, 53-55, 61, 72, 74, 75, 84, 88, 99, 101, 110, 115, 119, et un fragment polynucléotidique d'au moins 18 nucléotides contigus d'une telle séquence, dans lequel ledit agent de transfert est une composition de tensioactif organosilicone ou un composé organosilicone contenu dans celle-ci et conditionne ladite surface de ladite plante pour la perméation par ledit polynucléotide ARNdb, et ce par quoi la croissance, le développement ou l'aptitude à la reproduction de ladite plante est réduit(e) ou ladite plante est plus sensible à un herbicide inhibiteur d'EPSPS par rapport à une plante non traitée.

2. Procédé tel que revendiqué dans la revendication 1, dans lequel
(i) ladite plante est choisie dans l'ensemble constitué par *Amaranthus palmeri, Amaranthus rudis, Amaranthus graecizans, Amaranthus hybridus, Amaranthus lividus, Amaranthus spinosus, Amaranthus thunbergii, Amaranthus viridis, Lolium multiflorum, Lolium rigidum, Ambrosia artemisiifolia, Ambrosia trifida, Euphorbia heterophylla, Kochia scoparia, Abutilon theophrasti, Sorghum halepense, Chenopodium album, Commelina diffusa, Conyza canadensis* et *Digitaria sanguinalis* ; ou
(ii) ladite composition comprend encore
(a) ledit herbicide inhibiteur d'EPSPS ;
(b) un ou plusieurs herbicide(s) différent(s) dudit herbicide inhibiteur d'EPSPS ;
(c) un herbicide de type auxine ; ou
(d) de l'acide 3,6-dichloro-2-méthoxybenzoïque ou de l'acide 2,4-dichlorophénoxyacétique.

3. Procédé tel que revendiqué dans la revendication 1, dans lequel ledit polynucléotide ARNdb a une longueur d'au moins 21 nucléotides contigus ou ladite composition comprend une association quelconque de deux ou plus de deux desdits polynucléotides ARNdb.

4. Composition destinée à l'application topique sur une surface d'une plante, comprenant un polynucléotide ARNdb et un agent de transfert, dans laquelle ledit polynucléotide ARNdb est identique à ou complémentaire d'une séquence d'ARN d'une séquence de gène d'EPSPS choisie dans l'ensemble constitué par les séquences SEQ ID NO : 11, 18, 19, 21, 22, 53-55, 61, 72, 74, 75, 84, 88, 99, 101, 110, 115, 119, et un fragment polynucléotidique d'au moins 18 nucléotides contigus d'une telle séquence, dans laquelle ledit agent de transfert est une composition de tensioactif organosilicone ou un composé organosilicone contenu dans celle-ci et conditionne ladite surface de ladite plante pour la perméation par ledit polynucléotide ARNdb, et ce par quoi la croissance, le développement ou l'aptitude à la reproduction de ladite plante traitée par ladite composition est supprimé(e) ou retardé(e) ou ladite plante est plus sensible à un herbicide inhibiteur d'EPSPS par suite de ladite composition contenant un polynucléotide ARNdb, par rapport à une plante non traitée.

5. Composition selon la revendication 4, dans laquelle
(i) ledit polynucléotide ARNdb a une longueur d'au moins 21 nucléotides contigus ;
(ii) ledit polynucléotide ARNdb est choisi dans l'ensemble constitué par les séquences SEQ ID NO : 121-126, 137-140, 143-148, 213, 214, 231-240, 259-276, 281-308, 313-332, 345-356, 367-380, 383-442, 449-460, 479-496, 501-528, 533-550, 565-576, 591-600, 603-630, 635-642, 659-666, 687, 688, 709-718, 751, 752, 755-760, 765, 766, 771-784, 799-804, 811-818, 857-872, 893-898, 901, 902, 907-912, 915, 916, 921-930, 933, 934, 943-950, 955-958, 971, 972, 981-984, 989, 990, 993-1002, 1007, 1008, 1017, 1018, 1021, 1022, 1025-1032, 1035, 1036, 1041-1048, 1051-1056, 1059, 1060, 1071-1104, 1107-1154, 1159-1186, 1189-1290, 1293-1304, 1307-1322, 1325-1336, 1341-1344, 1347-1350, 1353-1472, 1477-1480, 1489-1492, 1497-1500, 1507-1522, 1527-1540, 1543-1546, 1551-1564, 1567-1584, 1587-1604, 1607-1678, 1681-1694, 1697-1702, 1707-1718, 1723-1738, 1741-1756, 1763-1792, 1797-1800, 1809-1824, 1827-1838, 1841-1856, 1859-1870, 1875-1878, 1881-1884, 1887-1932, 1935-1948, 1951-1956, 1961-1972, 1977-2000, 2003-2014, 2017-2032, 2035-2046, 2051-2054, 2057-2060, 2063-2146, 2149, 2150, 2161, 2162, 2167, 2168, 2187-2194, 2197, 2198, 2211-2256, 2259-2264, 2267, 2268, 2271-2276, 2283-2308, 2311-2388, 2391, 2392, 2395-2402, 2405, 2406, 2409, 2410, 2413, 2414, 2417-2426, 2429-2464, 2467, 2468, 2471-2476, 2483-2500, 2503-2514, 2517-2532, 2539-2550, 2557-2602, 2605-2608, 2611-2634, 2637-2640, 2643-2646, 2649, 2650, 2663-2730, 2735-2738, 2745-2774, 2779-2796, 2799-2820, 2823-2842, 2845-2854, 2857, 2858, 2863-2870, 2873-2880, 2887, 2888, 2891-2896, 2901-2904, 2911-2920, 2927, 2928, 2931-2936, 2941.-2944, 2951-2958, 2963-3000, 3019, 3020, 3043-3054, 3067-3074, 3085-3146, 3151, 3152, 3155-3158, 3161-3174, 3187-3194, 3203, 3204, et 3207-3216 ;
(iii) ledit polynucléotide ARNdb est choisi dans l'ensemble constitué par les séquences SEQ 1D NO : 3235, 3258, 3260, 3268, 3280, 3283, 3289, 3307, 3308, 3313, 3320, 3338, 3344, 3345, 3351, 3370, 3397, 3399, 3411, 3431, 3438, 3449, 3452, 3455, 3467, 3472, 3479, 3485, 3492, 3517, 3521, 3524, 3530, 3533, 3538 et 3542 ; ou
(iv) ladite composition comprend encore
(a) ledit herbicide inhibiteur d'EPSPS ;
(b) du glyphosate ;
(c) un co-herbicide ;
(d) un herbicide de type auxine ; ou
(e) de l'acide 3,6-dichloro-2-méthoxybenzoïque ou de l'acide 2,4-dichlorophénoxyacétique.

6. Procédé de réduction de l'expression d'un gène d'EPSPS chez une plante, comprenant : le fait d'appliquer topiquement sur une surface d'une plante une composition comprenant un polynucléotide ARNdb et un agent de transfert, dans lequel ledit polynucléotide ARNdb est identique à ou complémentaire d'une séquence d'ARN d'une séquence de gène d'EPSPS choisie dans l'ensemble constitué par les séquences SEQ ID NO : 11, 18, 19, 21, 22, 53-55, 61, 72, 74, 75, 84, 88, 99, 101, 110, 115, 119, et un fragment polynucléotidique d'au moins 18 nucléotides contigus d'une telle séquence, dans lequel ledit agent de transfert est une composition de tensioactif organosilicone ou un composé organosilicone contenu dans celle-ci et conditionne la surface de ladite plante pour la perméation par ledit polynucléotide ARNdb, et ce par quoi ladite expression dudit gène d'EPSPS est réduite, par rapport à une plante non traitée.

7. Procédé tel que revendiqué dans la revendication 6, dans lequel
(i) ledit polynucléotide ARNdb a une longueur d'au moins 21 nucléotides contigus.

8. Procédé d'identification de polynucléotides ARNdb utiles dans la modulation de l'expression d'un gène d'EPSPS lorsqu'ils sont appliqués sur une surface d'une plante, comprenant les étapes consistant à : a) fournir une pluralité de polynucléotides d'ARNdb qui comprennent une région identique à ou complémentaire d'au moins 18 nucléotides contigus d'une séquence d'ARN d'une séquence de gène d'EPSPS, choisie dans l'ensemble constitué par les séquences SEQ ID NO : 11, 18, 19, 21, 22, 53-55, 61, 72, 74, 75, 84, 88, 99, 101, 110, 115, et 119; b) appliquer topiquement sur ladite surface de ladite plante une composition comprenant un ou plusieurs desdits polynucléotides ARNdb et un agent de transfert ; et c) analyser ladite plante, ou un extrait végétal de celle-ci, à la recherche de la modulation de l'expression du gène d'EPSPS, dans lequel ledit agent de transfert est une composition de tensioactif organosilicone ou un composé organosilicone contenu dans celle-ci et conditionne la surface de ladite plante pour la perméation par ledit polynucléotide ARNdb, et ce par quoi la croissance, le développement ou l'aptitude à la reproduction de ladite plante traitée par ladite composition est supprimé(e) ou retardé(e) ou ladite plante est plus sensible à un herbicide inhibiteur d'EPSPS par suite de ladite composition contenant un polynucléotide ARNdb, par rapport à une plante non traitée.

9. Procédé tel que revendiqué dans la revendication 8, dans lequel
(i) ladite plante est choisie dans l'ensemble constitué par *Amaranthus palmeri, Amaranthus rudis, Amaranthus graecizans, Amaranthus hybridus, Amaranthus lividus, Amaranthus spinosus, Amaranthus thunbergii, Amaranthus viridis, Lolium multiflorum, Lolium rigidum, Ambrosia artemisiifolia, Ambrosia trifida, Euphorbia heterophylla, Kochia scoparia, Abutilon theophrasti, Sorghum halepense, Chenopodium album, Commelina diffusa, Conyza canadensis* et *Digitaria sanguinalis* ; or
(ii) ladite expression du gène d'EPSPS est réduite par rapport à une plante non traitée par ladite composition.

10. Composition chimique agricole destinée à l'application topique sur une surface d'une plante, comprenant un mélange d'un polynucléotide ARNdb, d'un agent de transfert, de glyphosate, et d'un co-herbicide, dans laquelle ledit polynucléotide ARNdb est identique à ou complémentaire d'une séquence d'ARN d'une séquence de gène d'EPSPS choisie dans l'ensemble constitué par les séquences SEQ ID NO : 11, 18, 19, 21, 22, 53-55, 61, 72, 74, 75, 84, 88, 99, 101, 110, 115, 119, et un fragment polynucléotidique d'au moins 18 nucléotides contigus d'une telle séquence, dans laquelle ledit agent de transfert est une composition de tensioactif organosilicone ou un composé organosilicone contenu dans celle-ci et conditionne la surface de ladite plante pour la perméation par ledit polynucléotide ARNdb, et ce par quoi la croissance, le développement ou l'aptitude à la reproduction de ladite plante est supprimé(e) ou retardé(e) ou ladite plante est plus sensible à un herbicide inhibiteur d'EPSPS par suite de ladite composition contenant un polynucléotide ARNdb, par rapport à une plante non traitée.

11. Composition chimique agricole selon la revendication 10, dans laquelle ledit co-herbicide est choisi dans l'ensemble constitué par les herbicides de type amide, les herbicides arsénicaux, les herbicides de type benzothiazole, les herbicides de type benzoylcyclohexanedione, les herbicides de type alkylsulfonate de benzofuranyle, les herbicides de type carbamate, les herbicides de type cyclohexène-oxime, les herbicides de type cyclopropylisoxazole, les herbicides de type dicarboximide, les herbicides de type dinitroaniline, les herbicides de type dinitrophénol, les herbicides de type éther diphénylique, les herbicides de type dithiocarbamate, les herbicides aliphatiques halogénés, les herbicides de type imidazolinone, les herbicides inorganiques, les herbicides de type nitrile, les herbicides organophosphorés, les herbicides de type oxadiazolone, les herbicides de type oxazole, les herbicides de type phénoxy, les herbicides de type phénylènediamine, les herbicides de type pyrazole, les herbicides de type pyridazine, les herbicides de type pyridazinone, les herbicides de type pyridine, les herbicides de type pyrimidinediamine, les herbicides de type pyrimidinyloxybenzylamine, les herbicides de type ammonium quaternaire, les herbicides de type thiocarbamate, les herbicides de type thiocarbonate, les herbicides dérivés de thiourée, les herbicides de type triazine, les herbicides de type triazinone, les herbicides de type triazole, les herbicides de type triazolone, les herbicides de type triazolopyrimidine, les herbicides à base d'uracile et les herbicides dérivés d'urée.

12. Composition chimique agricole destinée à l'application topique sur une surface d'une plante, comprenant un mélange d'un polynucléotide ARNdb, d'un agent de transfert, de glyphosate, et d'un pesticide, dans laquelle ledit polynucléotide ARNdb est identique à ou complémentaire d'une séquence d'ARN d'une séquence de gène d'EPSPS choisie dans l'ensemble constitué par les séquences SEQ ID NO : 11, 18, 19, 21, 22, 53-55, 61, 72, 74, 75, 84, 88, 99, 101, 110, 115, 119, et un fragment polynucléotidique d'au moins 18 nucléotides contigus d'une telle séquence, dans laquelle ledit agent de transfert est une composition de tensioactif organosilicone ou un composé organosilicone contenu dans celle-ci et conditionne la surface de ladite plante pour la perméation par ledit polynucléotide ARNdb, et ce par quoi la croissance, le développement ou l'aptitude à la reproduction de ladite plante traitée par ladite composition est supprimé(e) ou retardé(e) ou ladite plante est plus sensible à un herbicide inhibiteur d'EPSPS par suite de ladite composition contenant un polynucléotide ARNdb, par rapport à une plante non traitée.

13. Composition chimique agricole selon la revendication 12, dans laquelle ledit pesticide est choisi dans l'ensemble constitué par les insecticides, fongicides, nématicides, bactéricides, acaricides, régulateurs de croissance, chimiostérilisants, substances sémiochimiques, répulsifs, attractants, phéromones, phagostimulants et biopesticides.
